# EUROPEAN PATENT SPECIFICATION

(11) **EP 0 831 892 B1**
(45) Date of publication and mention of the grant of the patent: **10.06.2009**
(21) Application number: 96913166.3
(22) Date of filing: 25.04.1996
(51) Int. Cl.: A61K 38/46, A61K 39/02, C12N 9/14, A61K 39/106

(54) **MULTIMERIC, RECOMBINANT UREASE VACCINE**
MULTIMERER, REKOMBINANTER UREASE IMPFSTOFF
VACCIN D'UREASE RECOMBINANTE ET MULTIMERE

(30) Priority: 28.04.1995 US 431041; 06.12.1995 US 568122
(43) Date of publication of application: 01.04.1998
(73) Proprietor: Merieux-OraVax SNC, 69007 Lyon (FR)
(72) Inventor: LEE, Cynthia, K., Needham, MA 02192 (US); MONATH, Thomas, P., Harvard, MA 01451 (US); ACKERMAN, Samuel, K., Weston, MA 02193 (US); THOMAS, William, D., Jr., Somerville, MA 02114 (US); SOMAN, Gopalan, Belmont, MA 02178 (US); KLEANTHOUS, Harold, Newtonville, MA 02160 (US); WELTZIN, Richard, A., Lunenburg, MA 01462 (US); PAPPO, Jacques, Newton, MA 02168 (US); ERMAK, Thomas, Brookline, MA 02146 (US); GUIRAKHOO, Farshad, Melrose, MA 02176 (US); BHAGAT, Hitesh, Wayland, MA 01778 (US); SUSSMAN, Ilene, Newton, MA 02159 (US)
(74) Representative: Vossius, Volker
(86) International application number: PCT/US1996/005800
(87) International publication number: WO 1996/033732

(56) References cited:
- WO-A-94/09823
- WO-A-94/26901
- WO-A-95/22987
- WO-A-96/31235
- HU L-T ET AL: "PURIFICATION OF RECOMBINANT HELICOBACTER-PYLORI UREASE APOENZYME ENCODED BY UREA AND UREB" INFECTION AND IMMUNITY, vol. 60, no. 7, 1992, pages 2657-2666, XP002188928 ISSN: 0019-9567
- MICHETTI P ET AL: "Immunization of BALB/c mice against Helicobacter felis infectio with Helicobacter pylori urease" GASTROENTEROLOGY, SAUNDERS, PHILADELPHIA, PA,, US, vol. 107, no. 4, October 1994 (1994-10), pages 1002-1011, XP002120881 ISSN: 0016-5085
- KLEANTHOUS H ET AL: "Oral Immunization with recombinant Helicobacter pylori urease Apoenzyme in the Treatment of Helicobacter Infection." ABSTRACTS OF THE GENERAL MEETING OF THE AMERICAN SOCIETY FOR, vol. 95, 1995, page 183 XP002188929 95th General Meeting of the American Society for Microbiology;Washington, D.C., USA; May 21-25, 1995, 1995 ISSN: 1060-2011
- RAUWS E A J ET AL: "CURE OF DUODENAL ULCER ASSOCIATED WITH ERADICATION OF HELICOBACTER-PYLORI" LANCET (NORTH AMERICAN EDITION), vol. 335, no. 8700, 1990, pages 1233-1235, XP002188930 ISSN: 0099-5355
- DATABASE BIOSIS [Online] BIOSCIENCES INFORMATION SERVICE, PHILADELPHIA, PA, US; 1993 PHILLIPS K ET AL: "Antibacterial action of the urease inhibitor acetohydroxamic acid on Helicobacter pylori." Database accession no. PREV199396037419 XP002188931 & JOURNAL OF CLINICAL PATHOLOGY (LONDON), vol. 46, no. 4, 1993, pages 372-373, ISSN: 0021-9746
- FERRERO R L ET AL: "RECOMBINANT ANTIGENS PREPARED FROM THE UREASE SUBUNITS OF HELICOBACTER SPP.: EVIDENCE OF PROTECTION IN A MOUSE MODEL OF GASTRIC INFECTION" INFECTION AND IMMUNITY, AMERICAN SOCIETY FOR MICROBIOLOGY. WASHINGTON, US, vol. 62, no. 11, 1 November 1994 (1994-11-01), pages 4981-4989, XP002011225 ISSN: 0019-9567
- DATABASE MEDLINE [Online] Accession number NLM1741153, 1992 VARGA ET AL: "HELICOBACTER PYLORI ALLERGY" XP002196733 & ORVOSI HETILAP, vol. 133, no. 6, 9 February 1992 (1992-02-09), pages 359-361,
- TELFORD J L ET AL: "UNRAVELLING THE PATHOGENIC ROLE OF HELICOBACTER PYLORI IN PEPTIC ULCER: POTENTIAL NEW THERAPIES AND VACCINES" TRENDS IN BIOTECHNOLOGY, ELSEVIER PUBLICATIONS, CAMBRIDGE, GB, vol. 12, no. 10, 1 October 1994 (1994-10-01), pages 420-426, XP000483385 ISSN: 0167-7799
- DATABASE BIOSIS [Online] BIOSCIENCES INFORMATION SERVICE, PHILADELPHIA, PA, US; 1994 TAPPUOLI R ET AL: "Pathogenesis of Helicobacter pylori and perspectives of vaccine development against an emerging pathogen." Database accession no. PREV199598093977 XP002196734 & BEHRING INSTITUTE MITTEILUNGEN, no. 95, 1994, pages 42-48, ISSN: 0301-0457
- DATABASE BIOSIS [Online] BIOSCIENCES INFORMATION SERVICE, PHILADELPHIA, PA, US; 1990 OTTO G ET AL: "ERADICATION OF HELICOBACTER-MUSTELAE FROM THE FERRET STOMACH AN ANIMAL MODEL OF HELICOBACTER-PYLORI CHEMOTHERAPY" Database accession no. PREV199090036768 XP002196735 & ANTIMICROBIAL AGENTS AND CHEMOTHERAPY, vol. 34, no. 6, 1990, pages 1232-1236, ISSN: 0066-4804
- GASTROENTEROLOGY, April 1993, Vol. 104, No. 4, DAVIN C. et al., "H. Pylori Urease Elicits Protection Against H. Felis in Mice", page A1035.
- LANCET, 21 July 1990, Vol. 336, PALLEN M.J. et al., "Vaccination Against Helicobacter Pylori Urease", pages 186-187.
- GASTROENTEROLOGY, vol. 104, no. 4, 1993, page A1035,

## Description

### Background of the Invention

This invention relates to compositions for preventing and/or treating *Helicobacter* infection.

*Helicobacter* is a genus of spiral, gram-negative bacteria which colonize the gastrointestinal tracts of mammals. Several species colonize the stomach, most notably, *H. pylori, H*. *heilmanii*, *H*. *felis,* and *H*. mustelae. Although *H. pylori* is the species most commonly associated with human infection, *H*. *heilmanii* and *H. felis* have also been found to infect humans, but at lower frequencies than *H. pylori.*

*Helicobacter* infects over 50% of adult populations in developed countries, and nearly 100% in developing countries and some Pacific rim countries, making it one of the most prevalent infections of humans worldwide. Infection with *H. pylori* results in chronic stomach inflammation in all infected subjects, although the clinical gastroduodenal diseases associated with *Helicobacter* infection generally appear from several years to several decades after the initial infection. *H*. *pylori* is the causative agent of most peptic ulcers and chronic superficial (type B) gastritis in humans. *H*. *pylori* infection is also associated with atrophy of the gastric mucosa, gastric adenocarcinoma, and non-Hodgkin's lymphoma of the stomach (see, *e*.*g*., Blaser, J. Infect. Dis. 161:626-633, 1990; Scolnick et al., Infect. Agents Dis. 1:294-309, 1993; Goodwin et al., Helicobacter pylori, Biology and Clinical Practice, CRC Press, Boca Raton, FL, 465 pp, 1993; Northfield et al., Helicobacter pylori, Infection, Kluwer Acad. Pub., Dordrecht, 178 pp, 1994).

WO 94/09823 discloses immunisation with Helicobacter urease or urease subunits.

### Summary of the Invention

We have shown that vaccine compositions containing multimeric, recombinant *Helicobacter* urease are effective in preventing and treating *Helicobacter* infection. In addition, we have shown that a composition containing *H. pylori* urease and a mucosal adjuvant, in combination with an antibiotic and a bismuth salt, is more effective at treating *Helicobacter* infection than any of these components alone, or any combinations of three or less of these components.

Accordingly, in a first aspect, the invention features a vaccine for inducing an immune response to *Helicobacter* (*e*.*g*., *H. pylori*) in a patient (*e*.*g*., a human patient) that (a) is at risk of developing, but does not have, a *Helicobacter* infection (a "protective" immune response), or (b) is already infected by *Helicobacter* (a "therapeutic" immune response). This vaccine contains multimeric complexes of recombinant, enzymatically inactive *Helicobacter* (*e.g., H. pylori*) urease, and a pharmaceutically acceptable carrier or diluent (*e*.*g*., sterile water or a 2% weight/volume sucrose solution). The vaccine may contain a multimeric complex containing eight Urease A subunits and eight Urease B subunits, a multimeric complex containing six Urease A subunits and six Urease B subunits, a multimeric complex containing four Urease A subunits and four Urease B subunits, or a mixture thereof. In addition the vaccine contains a mucosal adjuvant, which is the heat-labile enterotoxin of enterotoxigenic *Escherichia coli* (LT), a cholera toxin (CT), or a subunit or derivative thereof lacking toxicity but still having adjuvant activity (*e*.*g*., a fragment, mutant, or toxoid). Further, the multimeric complexes of recombinant, enzymatically inactive *Helicobacter* urease may be freeze-dried prior to use in the vaccines of the invention.

In a second aspect, the invention features the use of an immunologically effective amount of multimeric complexes of recombinant, enzymatically inactive *Helicobacter* urease and a mucosal adjuvant, wherein said mucosal adjuvant is the heat-labile enterotoxin of enterotoxigenic Escherichia coli, a cholera toxin, or fragment or mutant thereof still having adjuvant activity, for the preparation of a vaccine for inducing a mucosal immune response to *Helicobacter* in a patient by mucosal administration.

In this aspect of the invention, a composition containing an immunogenically effective amount of multimeric complexes of recombinant, enzymatically inactive *Helicobacter* (*e*.*g*., *H. pylori*) urease is administered to a mucosal surface (*e*.*g*., an oral or intranasal surface; or to a rectal surface by, *e*.*g.*, anal suppository) of the patient, *e.g.* a human. The composition may contain a multimeric complex containing eight Urease A subunits and eight Urease B subunits, a multimeric complex containing six Urease A subunits and six Urease B subunits, a multimeric complex containing four Urease A subunits and four Urease B subunits, or a mixture thereof. The composition may be administered without gastric neutralization by, *e*.*g*., sodium bicarbonate.

In addition to the multimeric complexes of recombinant, enzymatically inactive *Helicobacter* urease, the composition used in this aspect of the invention further contains a mucosal adjuvant which is the heat-labile enterotoxin of enterotoxigenic *Escherichia* coli (LT), a cholera toxin (CT), or a subunit or derivative thereof lacking toxicity but still having adjuvant activity (*e*.*g*., fragments, mutants, or toxoids having adjuvant activity).

Further, the multimeric complexes of recombinant, enzymatically inactive *Helicobacter* urease in the composition may be freeze-dried prior to use in this method.

By "vaccine" is meant a composition containing at least one antigen (*e*.*g*., an antigen from a pathogenic microorganism, such as *H. pylori*) which, when administered to a patient, elicits or enhances an immune response to the antigen that is effective in the prevention of disease (*e*.*g*., disease caused by infection by the microorganism), or in the treatment of a pre-existing disease.

By "immunogenically effective amount" is meant an amount of an antigen (*e*.*g*., multimeric complexes of recombinant, enzymatically inactive *Helicobacter* urease, or any of the other antigens listed above) which is effective to elicit an immune response (*e*.*g*., a humoral or a mucosal immune response) when administered to a patient, such as a human patient.

By "*Helicobacter*" is meant any bacterium of the genus *Helicobacter*, particularly a *Helicobacter* which infects humans (*e*.*g*., *H. pylori*). By "urease," is meant an enzyme (*e*.*g*., *H. pylori* urease) which catalyzes the conversion of urea into ammonium hydroxide and carbon dioxide.

By "multimeric complex" is meant a macromolecular complex of polypeptides (*e*.*g*., urease polypeptides). The polypeptides may be associated in the complex by a variety of intermolecular interactions, such as covalent bonds (*e*.*g*., disulfide bonds), hydrogen bonds, and ionic bonds.

By "mucosal adjuvant" is meant a compound (*e*.*g*., an immunomodulator) which non-specifically stimulates or enhances a mucosal immune response (*e*.*g*., production of IgA antibodies). Administration of a mucosal adjuvant in combination with an immunogenic composition facilitates the induction of a mucosal immune response to the antigen in the composition.

By "antibiotic" is meant a compound derived from a mold or bacterium that inhibits the growth of other microorganisms.

Other features and advantages of the invention will be apparent from the following detailed description and from the claims.

### Detailed Description

The drawings are first described.

### Drawings

**Fig. 1** is a diagram showing the restriction enzyme map of the 2.5 kb PCR product cloned from pSCP1. The numbers above the map indicate nucleotide positions using the first base pair (bp) of BL1 as nucleotide #1. Locations of *ureA* and *ureB* genes and the direction of transcription of these genes are shown. The locations of restriction enzyme cleavage sites are shown below the genes. The numbers next to the name of each restriction enzyme indicate the locations of the first base pair in the recognition sequence for the enzyme indicated.

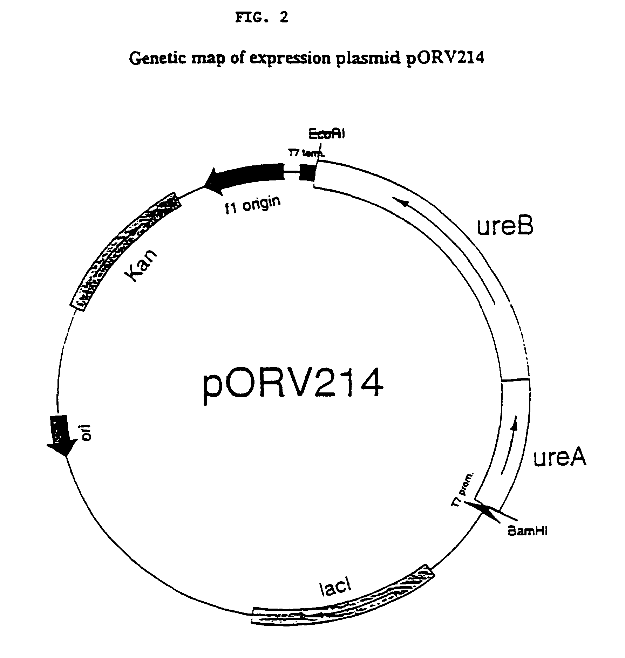

**Fig. 2** is a diagram of the genetic map of the expression plasmid pORV214. The *ureA* and *ureB* genes are shown as open bars with arrows. The solid arrow and solid bar represent the T7 promoter and terminator sequences, respectively. Thin lines represent pBR322 DNA. The phage and plasmid origins of replication on the plasmid are shown by large shaded arrows, denoted "f1 origin" and "ori," respectively, and indicate the direction of DNA synthesis. The genes encoding kanamycin resistance (kan) and the lactose repressor (*lacI*) are shown as shaded bars. The *BamH*I and *EcoR*I sites used for cloning the PCR product are indicated. Arrows within bars representing *lacI*, *ureA*, *ureB*, and *kan* genes indicate the direction of transcription.

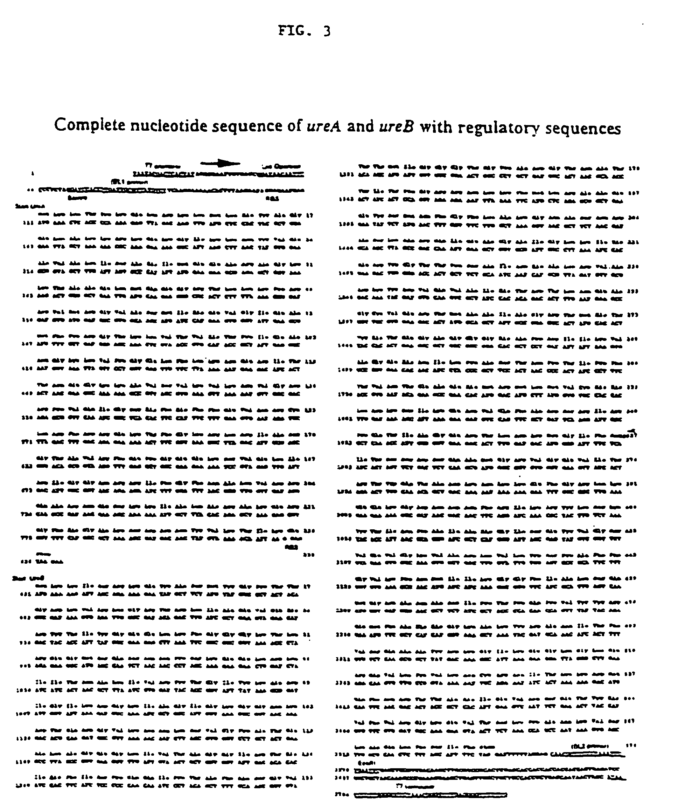

**Fig. 3** is a schematic representation of the nucleotide sequence of the *H*. *pylori* locus encoding the *ureA* and *ureB* genes, as well as the transcriptional regulatory sequences from pORV214 (SEQ ID NO:1). The predicted amino acid sequences of *ureA* (SEQ ID NO:2) and *ureB* (SEQ ID NO:3) are shown above the DNA sequence. Numbers to the left of the sequence correspond to nucleotide positions and those on the right indicate amino acid positions. The sequences corresponding to the PCR primers are underlined and the *BamH*I and *EcoR*I sites used to clone the PCR product are indicated. The predicted transcriptional initiation site (G) and direction of transcription are shown with an arrow. The ribosomal binding site (RBS) is indicated. The sequences which regulate transcriptional initiation (T7 promoter and *lac* operator) and termination are underlined and labeled.
**Fig. 4** is a graph showing analytical size exclusion HPLC for recombinant urease.
**Fig. 5** is a series of graphs showing the bacterial score versus the levels of serum IgA, serum IgG, and fecal IgA antibodies from mice immunized with recombinant *H. pylori* urease and cholera toxin (CT).
**Fig. 6** is a series of graphs showing the bacterial score versus the levels of serum IgA, serum IgG, and fecal IgA antibodies from mice immunized with recombinant *H. pylori* urease and enterotoxigenic *E. coli* heat-labile toxin.
**Fig. 7** is a table showing the experimental protocol used for comparing intranasal and oral immunization routes for recombinant urease.
**Fig. 8** is a series of graphs showing the serum IgG (Serum G), serum IgA (Serum A), fecal IgA (Fecal A), and salivary IgA (Sal A) levels in groups of mice treated according to the protocol illustrated in Fig. 7.
**Fig. 9** is a table showing the experimental protocol used for comparing intranasal and intragastric immunization routes for recombinant urease.
**Fig. 10** is a series of graphs showing the results of urease tests carried out on the mice treated according to the protocol illustrated in Fig. 9.
**Fig. 11** is a graph showing that rUrease (10 µg) given by the intranasal route with CT (5 µg) is at least as effective as rUrease (25 µg) given by the oral route with CT (10 µg) in preventing infection with *H. felis.*
**Fig. 12** is a graph showing that immunization of mice with rUrease + 10 µg LT reduces or eradicates established *H. felis* infection. When these animals were reinfected with *H*. *felis,* they were protected against challenge.
**Fig. 13** is a graph showing the mean antibody response of mice 4 and 10 weeks after therapeutic immunization with either recombinant *H. pylori* urease and CT, or CT alone.
**Fig. 14** is a graph showing numbers of IgA antibody secreting cells in gastric mucosa of immunized and unimmunized mice before, and at intervals after, infection with *H*. *felis*.
**Fig. 15** is a graph showing the clearance of *H. felis* infection in mice previously immunized with recombinant *H. pylori* urease and CT.

### Cloning of the ureA and ureB genes

The structural genes encoding urease, *ureA*, and *ureB*, have been cloned (Clayton et al., Nucl. Acids. Res. 18:362, 1990; Labigne et al., J. Bacteriol. 173:1920-1931, 1991), and the recombinant urease encoded by these genes has been purified (Hu et al., Infect. Immun. 60:2657-2666, 1992). For use in the present invention, urease was cloned from a clinical isolate of *H. pylori* (CPM630) obtained from a clinical specimen provided by Dr. Soad Tabaqchali, St. Bartholomew's Medical College, University of London. A genomic DNA library of strain CPM630 was prepared in the lambda phage vector EMBL3. Plaques were screened for reactivity with rabbit anti-*Helicobacter* urease polyclonal antibody, and a single reactive plaque was isolated. This clone contained a 17 kb *Sal*I fragment that encoded the *ureA* and *ureB* genes. The 17 kb fragment was subcloned onto pUC18 and designated pSCP1. A 2.7 kb *Taq*I fragment was subcloned (pTCP3) and completely sequenced. The 2.7 kb *Taq*I fragment encoded both *ureA* and *ureB*.

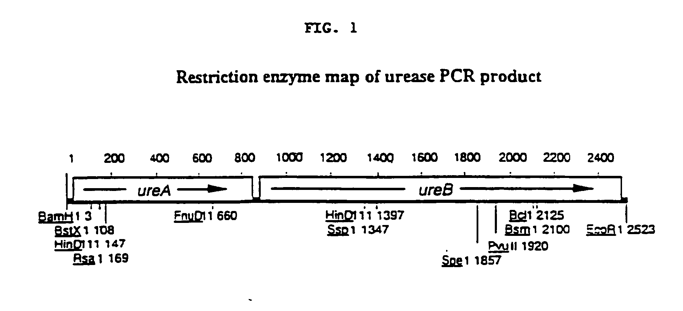

The primers BL1 (CGG GAT CCA CCT TGA TTG CGT TAT GTC T; SEQ ID NO:4) and BL2 (CGG AAT TCA GGA TTT AAG GAA GCG TTG; SEQ ID NO:5) were used to amplify and clone a 2.5 kb fragment from pSCP1. BL1 and BL2 correspond to nucleotides 2605-2624 of GenBank accession number M60398 (the BL1 primer) and nucleotides 2516-24998 of EMBL accession number X17079 (the BL2 primer). A restriction enzyme map of the 2.5 kb fragment PCR product is shown in Fig. 1. The 2.5 kb fragment contains the entire coding region of *ureA* and *ureB*, as well as translational start signals from *H. pylori* upstream of *ureA*.

### Expression of recombinant urease

The purified 2.5 kb PCR fragment containing the genes encoding *ureA* and *ureB* was digested with *EcoR*I and *BamH*I and inserted into the expression vector pET24+ (Novagen, Madison, Wisconsin) to produce the plasmid pORV214 (Fig. 2). pET24+ contains the colE1 origin of replication, the filamentous phage (fl) origin of replication for single strand rescue, and the kanamycin resistance gene of Tn903. The fragment was inserted downstream of the T7 promoter, which provides transcription initiation for the urease genes. Lactose operator (*lacO*) sequences are present between the T7 promoter and the cloning sites to provide inducible expression of the urease genes. A T7 transcription terminator sequence is located downstream of the cloning sites. The vector also contains the lactose repressor gene (*lacI*) to ensure complete repression of expression. Other sequences present in the vector are derived from pBR322, which served as the backbone for vector construction.

The initial ligation mixture, containing the 2.5 kb PCR *EcoR*I-*BamH*I fragment and the pET24+ vector digested with *EcoR*I and *BamH*I, was used to transform XL1-Blue (Stratagene, La Jolla, CA) prepared by the CaCl₂ method. XL1-Blue is an *E*. *coli* strain that does not express T7 RNA polymerase. Kanamycin resistant colonies were directly screened by PCR using urease specific primers. Plasmid DNA from several positive colonies was extracted using Qiagen minispin columns (Qiagen, Chatsworth, CA) and checked for the correct restriction digestion pattern.

Purified pORV214 DNA was used to transform the *E. coli* strain BL21-DE3 (Novagen, Madison, Wisconsin) prepared by the CaCl₂ method. BL21-DE3 is an *E. coli* B strain that is lysogenized with lambda phage DE3, a recombinant phage that encodes T7 RNA polymerase under the control of the *lavUV*5 promoter. BL21 is deficient in *lon* and *ompT* proteases, as well as the *hsdS_{B}* restriction/modification system and dcm DNA methylation. DNA was prepared from kanamycin resistant colonies with Qiagen mini spin columns and screened by restriction enzyme analysis using *BamH*I and *EcoR*I to confirm the presence of the plasmid. Urease expression was assessed by examination of BL21-DE3 (pORV214) cell lysates by SDS-PAGE and Western blot. Several positive clones had the correct restriction endonuclease digestion pattern and expressed urease.

A single clone containing pORV214 was selected, grown on LB plates containing kanamycin (50 µg/ml), harvested, and stored at -80°C in LB containing 50% glycerol. A research cell bank was prepared by growing a sample from the glycerol stock on LB plates containing kanamycin, selecting an isolated colony, and inoculating a LB broth culture containing kanamycin. This culture was grown to OD₆₀₀ of 1.0, pelleted, and resuspended in an equal volume of LB containing 50% glycerol. These research cell bank (RCB) aliquots (100 µl) were then stored at -80°C. A master cell bank (MCB) was similarly prepared using an isolated colony from the research cell bank, and a manufacturer's working cell bank (MWCB) was prepared using an isolated colony from the MCB.

The MCB and MWCB cells were viable, kanamycin resistant, and displayed a normal *E. coli* colony morphology. T7 RNA polymerase expression and lambda phage lysogeny was confirmed using appropriate tests, which are well known in the art. Urease expression was IPTG-inducible in the MCB and MWCB cells, as determined by examination of cultures grown in the presence of IPTG, and analysis of lysates from these cell cultures on SDS-PAGE. Production of 60 kD and 29 kD proteins (UreB and UreA, respectively) by the MCB and MWCB cells increased with the incubation time.

Plasmid DNA was isolated from the MCB and MWCB cells and was tested by restriction enzyme analysis, restriction fragment length polymorphism (RFLP), and DNA sequence analysis to confirm plasmid structure. The MCB contained a plasmid with the appropriate restriction endonuclease digestion pattern with no deletions or rearrangements of the plasmid. Likewise, there were no differences between the RFLP fingerprint of pSCP1 and the RFLP fingerprints of plasmid DNA from MCB and MWCB, indicating that the urease genes had not undergone detectable (> 50 bp) deletions or rearrangements in the cloning process or in the manufacture of the cell banks.

The coding regions of *ureA* and *ureB*, and the sequences of the promoter and termination regions of the plasmid isolated from MCB cells, were sequenced. Sequencing reactions were performed using the Di-Deoxy Cycle Sequencing Kit, according to the manufacturer's instructions (Applied Biosystems, Inc., Foster City, CA), using fluorescent-labeled dideoxynucleotides. The sequences of the *ureA* and *ureB* genes, with predicted protein sequences, as well as the DNA sequences of flanking regions are shown in Fig. 3.

### Large-scale production of recombinant urease

The fermentor(s) to be used was cleaned and sterilized according to approved procedures. The culture medium contained 24 g/L yeast extract, 12 g/L tryptone, 6-15 g/L glycerol, in RO/DI water. Since pORV214 was sufficiently stable in the absence of antibiotics, no antibiotics were present in the large-scale fermentation cultures. Antifoam was added and the unit was sterilized in place.

For production in the 40 liter fermentor, a vial of the MWCB was thawed and used to inoculate a four liter shaker flask containing one liter of LB broth (1% tryptone, 0.5% yeast extract, 1% NaCl) without antibiotics. The culture was shaken at 37°C for 16-24 hours. The inoculum was then transferred to the 40 liter fermentor (30 liter working volume) containing the production media described above. Fermentation was carried out with aeration and agitation until the cell density determined by OD₆₀₀ was approximately 8-10. Isopropyl β-D-thiogalactopyranoside (IPTG) was then added to a final concentration of 0.1 mM, and induction was allowed to proceed for 16-24 hours. The cells were harvested by centrifugation and the wet paste was aliquoted into polypropylene storage containers and stored at -80°C. For production at the 400 liter scale (300 liter working volume), the inoculum and culture in the 40 liter vessel were prepared as described above. When the 40 liter vessel reached an OD₆₀₀ of approximately 5.0, it was used to inoculate the 400 liter fermentor. The culture was further incubated to an OD₆₀₀ of 8-10. The culture was then induced, harvested, and stored, as is described above. This procedure required 2-3 generations more than the 40 liter process.

### Purification of recombinant H. pylori urease

Containers of cell paste produced by the fermentation processes described above were removed from storage, thawed at room temperature, and resuspended in 20 mM sodium phosphate/1 mM EDTA buffer, pH 6.8. The resuspended cells were disrupted by extrusion through a narrow orifice under pressure (Microfluidizer Cell Disruptor). The disrupted cells were centrifuged at 4°C to sediment cell debris and the supernatant containing soluble urease was collected.

A solution of 3 M sodium chloride was added to the cell supernatant to a final concentration of 0.1 M. The supernatant was then applied to a DEAE-Sepharose column equilibrated in 20 mM sodium phosphate/1 mM EDTA, and the pass-through was collected for further processing. The pass-through was diafiltered into 20 mM sodium phosphate/1 mM EDTA, pH 6.8, with a 100 kD cutoff membrane to remove low molecular weight contaminants and to reduce ionic strength.

This material was applied to a second DEAE-Sepharose column equilibrated in 20 mM sodium phosphate/1 mM EDTA. The pass-through was discarded and the column rinsed with 20 mM sodium phosphate/1 mM EDTA, pH 6.8. The bound material was eluted with approximately three column volumes of 0.1 M NaCl/1 mM β-mercaptoethanol. Effective elution of the bound urease was controlled by the volume and flow rate of the elution buffer.

The partially purified urease was diafiltered against 25 mM Tris-HCl, pH 8.6, and applied to a Q-Sepharose column. The column was washed with approximately two column volumes of the same buffer and the pass-through containing highly purified urease was collected. The pass-through from the Q-Sepharose step was then concentrated and diafiltered into 2% sucrose in water for injection (WFI), pH 7.5.

### Characterization of the antigenicity and subunit composition of purified recombinant H. pylori urease

To compare the antigenicity and subunit composition of recombinant *H. pylori* urease to native *H. pylori* urease, native *H*. *pylori* urease was purified and used as an antigen to produce polyclonal anti-urease antibodies, as well as mono-specific polyclonal anti-UreA, anti-UreB, and anti-urease holoenzyme antibodies.

Native *H. pylori* urease was purified using a modification of the procedure reported by Hu and Mobley (Infect. Immun. 58:992-998, 1990). *H. pylori* strain ATCC 43504 (American Type Culture Collection, Rockville, MD) was grown on Mueller-Hinton agar (Difco Laboratories, Detroit, MI) containing 5% sheep red blood cells (Crane Labs, Syracuse, NY) and antibiotics (5 µg/ml trimethoprim, 10 µg/ml vancomycin and 10 U/ml polymyxin B sulfate) (TVP, Sigma Chemical Co., St. Louis, MO). Plates were incubated 3-4 days at 37°C in 7% CO₂ and 90% humidity, and bacteria were harvested by centrifugation. The bacteria were suspended in water or 20 mM phosphate, 1 mM EDTA, 1 mM β-mercaptoethanol (pH 6.8) containing protease inhibitors, lysed by sonication and clarified by centrifugation. The clarified supernatant was mixed with 3 M sodium chloride to a final sodium chloride concentration of 0.15 M and passed through DEAE-Sepharose (Fast Flow). Active urease that passed through the column was collected, concentrated in a Filtron Macrosep 100 centrifugal filtration unit, and then passed through a Superose-12 or Superdex 200 size exclusion column. Size-exclusion chromatography was performed using Pharmacia FPLC pre-packed columns. The fractions containing urease activity were pooled, concentrated, and further purified by FPLC anion-exchange chromatography on a Mono-Q sepharose column prepacked by Pharmacia. The bound urease was eluted using a sodium chloride gradient. The fractions with urease activity were pooled and concentrated using Macrosep 100 centrifugal filters from Filtron Inc. For some lots, a final purification was achieved by analytical size-exclusion FPLC on Superose-12 columns.

Polyclonal antiserum to *H. pylori* urease was produced by immunizing three female New Zealand white rabbits with purified native *H. pylori* urease. The animals were pre-bled to confirm non-immune status and then immunized subcutaneously with 150 µg urease in complete Freund's adjuvant. Two booster doses of 150 µg each were administered subcutaneously 27 and 45 days later with Freund's incomplete adjuvant. After confirmation of the immune response by ELISA and Western blotting against purified urease, the animals were exsanguinated. The blood was clotted at 4°C overnight and serum was harvested by centrifugation. Serum IgG was purified by ammonium sulfate precipitation (50%) overnight at 4°C. The precipitate was resuspended in PBS and dialyzed to remove ammonium sulfate. The anti-urease titer of the IgG from each animal was found to be 1:10⁷ and the antibodies from the three animals were pooled. The protein concentration was determined to be 17.3 mg/ml. Aliquots of 0.2 ml each were prepared and stored at -80°C.

Mouse polyclonal ascites against *H. pylori* urease holoenzyme ("MPA3") was prepared by injecting five mice subcutaneously with 10 µg native urease holoenzyme in complete Freund's adjuvant on Day 0. The mice were boosted on Days 10 and 17, bled on day 24 to confirm anti-urease immune response, and boosted again on Day 26. On Day 28 the mice were injected intraperitoneally with Sarcoma 180 cells. A final intraperitoneal booster dose of 10 µg urease was given on Day 31, and ascitic fluids collected seven days later.

The ascitic fluids were incubated for two hours at room temperature and then at 4°C for 16 hours; clots were disrupted by vortexing and removed by centrifugation at 10,000 rpm for 10 minutes. After overnight incubation at 4°C in plastic tubes, the fluids formed solid clots. These were homogenized, diluted five-fold with PBS, and re-clarified by centrifugation at 10,000 rpm for 10 minutes. Thirty-six ml of diluted ascitic fluids were collected and frozen at -20°C in 300 µl aliquots. Western blot analyses confirmed that the antibody reacts with UreA and UreB subunits. An endpoint titer of 1:300,000 was achieved in ELISA against urease.

Mouse polyclonal ascites against *H. pylori* UreA ("MPA4") was prepared by injecting mice subcutaneously with native UreA subunit *H. pylori* urease isolated by electroelution from SDS-PAGE gels. Subsequent steps in the preparation of anti-*ureA* ascites were performed as described above for generation of antibodies against the holoenzyme.

Mouse polyclonal ascites against native *H. pylori* UreB subunit ("MPA6") was prepared by injecting mice subcutaneously with native UreB isolated by electroelution from SDS-PAGE gels. Subsequent steps in the preparation of anti-UreB ascites were performed as described above for generation of antibodies against the holoenzyme.

MAB71 is an IgA monoclonal antibody against *H. felis* urease which recognizes a protective epitope on the B subunit. Preparation of this antibody is described in Czinn et al., J. Vaccine 11(6):637-642, 1993, and the hybridoma cell line which produces it was deposited with the ATCC and designated deposit number HB 11514. The antibodies described above were used in Western blot experiments to characterize the purified recombinant *H. pylori* urease.

### SDS-PAGE and Western blot analysis of recombinant urease

Recombinant urease was first analyzed by SDS-PAGE (12.5%) run under reducing conditions. Two major protein bands (29 kD and 60 kD) and several lighter bands (approximately 38 kD) were evident. To identify the proteins in these bands, Western blots were performed using the anti-urease and anti-urease subunit antibodies described above. The 60 kD protein reacted with MPA3 (anti-urease holoenzyme) and MPA6 (anti-UreB), but not with MPA4 (anti-UreA). The 29 kD protein reacted with MPA3 (anti-urease holoenzyme) and MPA4 (anti-UreA), but not with MPA6 (anti-UreB). The lighter 38 kD band reacted with MPA3 anti-urease holoenzyme) and MPA6 (anti-UreB), indicating that this protein is a portion of UreB.

Two faint high molecular weight (>150 kD) bands were evident in SDS-PAGE gels. Both bands reacted faintly with antibodies to both UreA and UreB, indicating that a minor portion of recombinant urease subunits form covalent units resistant to sulfhydryl reduction under the conditions used. No other protein bands were apparent in Coomassie-stained gels. Thus, all proteins detected in the purified product are either UreA, UreB, or a derivative of UreA or UreB.

The wet Coomassie-stained SDS-PAGE gel was scanned using an Ultroscan XL laser densitometer and a Gel Scan XL software program (Pharmacia-LKB Biotechnology, Piscataway, NJ). The densitometry data were consistent with a 1:1 molar ratio of the UreA:UreB subunits, as expected from the structure of native *H. pylori* urease. UreA and UreB accounted for more than 95% of the total protein present in the purified urease preparation. The estimated average value for the combined UreA + UreB peaks was 95.2% ± 1.2%.

### Analytical size-exclusion HPLC of purified recombinant urease

The purity and molecular composition of purified recombinant urease was determined by analytical size-exclusion high performance liquid chromatography. Chromatography was performed using a Beckman System Gold HPLC consisting of Pump 126, diode array dual wavelength detector 168, System Gold Software Version V7.11, Progel-TSK G4000SWXL (5 mm x 30 cm i.d.) column (Beckman Instruments, Brea, California), and SWXL guard column from Supel Co. Chromatography was performed under isocratic conditions using 100 mM phosphate, 100 mM sodium chloride buffer, pH 7.0. The column was calibrated using molecular weight markers from Pharmacia-LKB.

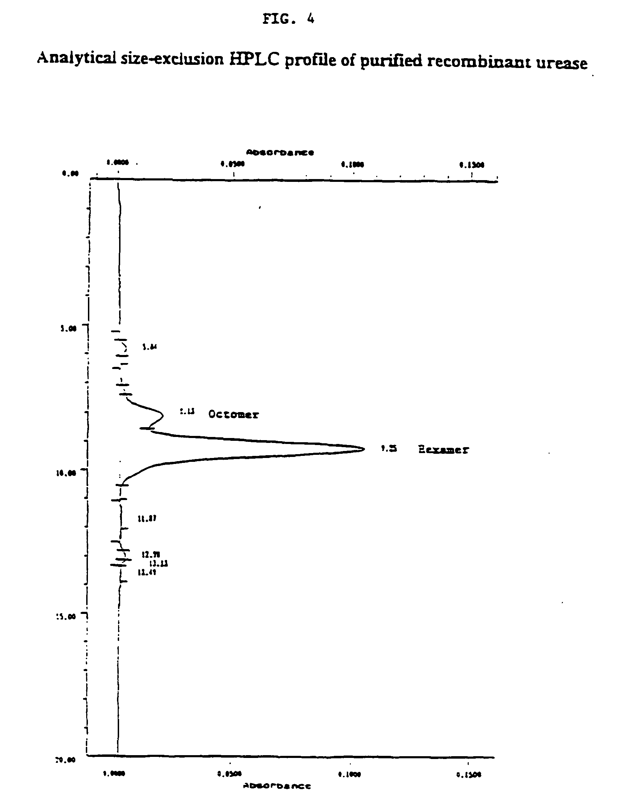

A typical HPLC separation profile of representative purified bulk sample is shown in Fig. 4. The purified urease product shows a prominent protein peak with a retention time between that of thyroglobulin (MW 669 kD) and ferritin (MW 440 kD). An apparent molecular weight of 550-600 kD was estimated based on a series of runs. This peak was tentatively designated as hexameric urease. The area of this hexameric urease was at least 70% of the total protein in different lots of product.

A second prominent protein peak with a lower retention time (higher molecular weight) was also detected. The area of this peak ranged from 5-20%. This peak, with a molecular weight >600 kD, was designated as octomeric urease. The total area of the octomeric plus hexameric urease peaks was over 90%.

These two characteristic peaks were isolated for further characterization. The two peaks were purified by HPLC from a preparation of reconstituted, freeze-dried urease and stored at 4°C for over one week. During storage for this period of time, octomeric urease increases while the hexameric form decreases. The separated peak fractions were analyzed by reducing SDS-PAGE and ELISA reactivity with monoclonal and polyclonal antibodies to urease. On SDS-PAGE, both peaks showed urease A and B bands in comparable ratios. In addition, both showed nearly identical immunoreactivity in ELISA with polyclonal anti-urease holoenzyme antibodies (MPA3) and a monoclonal anti-UreB antibody (MAB71).

### Enzymatic (urea hydrolytic) activity of recombinant urease

Three methods were used to investigate the enzymatic activity of recombinant urease: urease-specific silver staining following electrophoresis, the pH-sensitive phenol red urea broth assay, and direct detection of ammonia. Urease-specific silver staining, described by deLlano et al. (Anal. Biochem. 177:37-40, 1989), is based on the reaction of urease with urea to produce ammonia. The reaction leads to a localized increase in pH which facilitates a photographic redox reaction leading to disposition of metallic silver. Enzymatic activity of native *H*. *pylori* urease was detected with only a 1.0 µg sample. In contrast, 20 µg purified recombinant urease exhibited no urease activity.

The pH-sensitive phenol red broth assay, which is well known in the art, is based on a change in pH due to ammonia generation as a result of urea hydrolysis. Urease activity was demonstrated with as little as 0.2 µg/ml purified *H. pylori* urease. In contrast, no urease activity was associated with purified recombinant urease, even at concentrations up to 750 µg/ml.

Direct estimation of ammonia produced by hydrolysis of urea by urease was quantitated using Nesslers' reagent (Koch et al., J. Am. Chem. Society 46:2066-2069, 1924). Urease activity of native *H. pylori* urease was detectable at a concentration of 1 µg/ml. No activity was detected in assays containing up to 500 µg/ml purified recombinant urease.

Based on the results of the urease-specific silver staining assay, the pH-sensitive phenol red broth assay, and direct estimation of ammonia, there is no detectable urease activity in the recombinant urease product.

### Protective and therapeutic efficacy of purified recombinant urease

The *H. felis*-mouse model was used to test the efficacy of recombinant *H. pylori* urease in the prevention and treatment of *Helicobacter* infection. In this model, colonization of the stomach is readily established and is accompanied by gastric inflammation. This animal model is a well established system for the study of *Helicobacter*, and has been used extensively in laboratory investigations of the pathogenesis and treatment of *Helicobacter*-induced disease (see, *e*.*g*., Fox et al., Infect. Immun. 61:2309-2315, 1993; Goodwin and Worsley, Helicobacter pylori, Biology and Clinical Practice, CRC Press, Boca Raton, FL, 465 pp, 1993). Antigenic cross-reactivity between *H*. *pylori* and *H*. *felis* ureases allows use of the human vaccine candidate of the invention, recombinant *H. pylori* urease (rUre), to immunize animals infected, or subsequently challenged, with *H. felis.*

Both germ-free and conventional mice are susceptible to infection by *H. felis,* and develop life-long infection of the gastric epithelium, characterized by infiltration of inflammatory cells (Fox *et al*., *supra*). Dose response studies indicated that 100% of Swiss-Webster specific pathogen-free (SPF) mice become infected after a single oral challenge with 10⁴ *H. felis.* Unless otherwise specified, a single dose of 10⁷ was used to infect mice prior to therapeutic immunization or challenge mice after prophylactic immunization. A challenge of ∼10³ times the infectious dose (I.D.₉₀) with this *Helicobacter* represents a severe test of immunity.

### Assays for gastric infection

Several methods were used to detect *Helicobacter* in gastric tissue, including measurement of gastric urease activity, histologic examination, and culture of gastric tissue. Gastric urease activity was measured both qualitatively (presence or absence) and quantitatively. In the qualitative assay, stomachs were divided longitudinally into two halves from the gastroesophageal sphincter to the pylorus. One longitudinal piece, representing approximately 1/4 of the stomach, was placed in 1 mL of urea broth (0.1 g yeast extract, 0.091 g monopotassium phosphate, 0.095 g disodium phosphate, 20 g urea, and 0.1 g/L phenol red, pH 6.9). A distinctive color change (due to hydrolysis of urea by the enzyme, production of ammonia, and increased pH) after four hours incubation at room temperature indicated a positive result. For quantitative determinations, urease activity was determined by measuring absorbance at 550 nm of clarified urea broth incubated with whole stomach sections for 4 hours. This assay can detect as few as 1-2 x 10⁴ *H. felis*/0.1 g stomach tissue. This assay provides the same sensitivity as commercially available urease test kits used for human samples. Commercial kits have proven to be 100% specific and 90-92% sensitive compared to biopsy/histology (Szeto et al., Postgrad. Med. J. 64:935-936, 1988: Borromeo et al. J. Clin. Pathol. 40:462-468, 1987).

Quantitative gastric urease assays by spectrophotometric measurement of A₅₅₀ were slightly more sensitive than visual determinations, and allowed estimation of the severity of infection. The cut-off value for a negative urease assay was defined as 2 standard deviations above the mean A₅₅₀ for unchallenged/uninfected mice. The cut-off for a positive gastric urease assay was defined by 2 standard deviations below the mean A₅₅₀ of unimmunized/challenged mice. Individual animals with low-grade infections had values intermediate between the negative and positive cut-offs. Visual grading of the urease response identified 11/12 (92%) of the positive samples.

Histologic examination was performed by fixing stomach tissue in 10% formalin. The tissue was then embedded in paraffin, sectioned and stained with a modified Warthin-Starry silver stain (Steiner's stain; Garvey, et al. Histotechnology 8:15-17, 1985) to visualize *H. felis,* and by hematoxylin and eosin stain to assess inflammatory responses in the tissue. Stained sections were examined by an experienced pathologist blinded to the specimen code.

A semi-quantitative grading system was used to determine the number of bacteria and intensity of inflammation. The system used is a modification of the widely-accepted Sydney System for histological characterization of gastritis in humans (Price, Gastroenterol. Hepatol. 6:209-222, 1991). Full-thickness mucosal sections were examined for the intensity of inflammation (increase in lymphocytes, plasma cells, neutrophils and presence of lymphoid follicles) and depth of infiltration of these cells and graded on a scale of 0-4+. Grading the density of *H. felis* was accomplished by counting the number of bacterial cells with typical spiral morphology in an entire longitudinal section of gastric antrum (or corpus, if specified). Grades were assigned according to a range of bacteria observed (0 = none; 1+ = 1-20 bacteria; 2+ = 21-50 bacteria; 3+ = 51-100 bacteria; and 4+ = >100 bacteria).

### Route of immunization

The effect of the route of administration upon the efficacy of the vaccine of the invention was examined in the mouse infection model. Six to eight week-old female specific pathogen free (SPF) Swiss-Webster mice were immunized with 200 µg recombinant *H. pylori* urease, either with or without 0.24 M NaHCO₃. The recombinant urease was co-administered with 10 µg of cholera toxin (CT) as a mucosal adjuvant in all animals. Intragastric (IG) immunization was performed by delivering the antigen in 0.5 ml through a 20-gauge feeding needle to anesthetized animals. Oral immunization was performed by delivering the antigen in a 50 µl volume via a pipette tip to the buccal cavity of unanesthetized animals. For parenteral immunization, mice received 10 µg recombinant urease subcutaneously. Freund's complete adjuvant was used in the first subcutaneous immunization and Freund's incomplete adjuvant was used in subsequent boosters. For all routes of administration, a total of four doses of vaccine were administered at seven day intervals. Mice were challenged with 10⁷ *H. felis* two weeks after the final vaccine dose, and necropsied two weeks after challenge. Gastric *H. felis* infection was detected by urease activity and histology. Protection in an individual mouse was defined by a negative urease assay and by a 0 or 1+ bacterial score by histology.

Oral and intragastric administration of recombinant urease provided significant protection to challenge with *H. felis* (86-100%) (Table 1). Oral administration was effective both with and without coadministration of NaHCO₃ with the recombinant urease. Intragastric administration was more effective when the recombinant urease was co-administered with NaHCO₃. Parenteral injection of the vaccine antigen was least effective. Immunized mice had significantly lower numbers of bacteria in gastric tissue after challenge than unimmunized controls. IgA antibody responses in serum and secretions were highest in mice immunized by the oral route. IgA antibodies were not elicited by parenteral immunization.

**Table 1**

| Recombinant *H. pylori* urease protects mice from challenge with *H*. *felis* after mucosal but not parental immunization | | | | | |
|---|---|---|---|---|---|
| **VACCINE** | **ROUTE OF IMMUNIZATION** | **ADJUVANT** | **BICARBONATE^{a}** | **% PROTECTED (# PROTECTED/TOTAL)** | |
| | | | | **UREASE ASSAY** | **HISTOLOGY^{b}** |
| PBS | oral | CT | No | 0 (0/8) | 0 (0/7) |
| 200 µg rUre | oral | CT | No | 100 (8/8)* | 100 (7/7)* |
| 200 µg rUre | oral | CT | Yes | 100 (7/7)* | 86 (6/7)* |
| 200 µg rUre | intragastric | CT | No | 75 (6/8)* | 38 (3/8) |
| 200 µg rUre | intragastric | CT | Yes | 100 (7/7)* | 100 (7/7)* |
| 10 µg rUre | subcutaneous | Freund's | NA | 38 (3/8) | 25 (2/8) |

| | | | | | |
|---|---|---|---|---|---|
| ^{a} 0.24 M sodium bicarbonate was administered with vaccine and adjuvant. ^{b} Percent protected (number mice with 0-1+ bacterial score/number tested). ^{*} p<0.01, Fisher's exact test, compared to mice given CT alone. | | | | | |

The effect of immunization route upon the anti-urease antibody response was examined in mice. Swiss-Webster mice were immunized four times at ten day intervals with either: 1) 200 µg recombinant purified *H*. *pylori* urease with 10 µg CT, either with or without NaHCO₃, by oral administration; 2) 200 µg recombinant purified *H. pylori* urease and 10 µg CT with NaHCO₃, by intragastric administration; or 3) 10 µg recombinant purified *H. pylori* urease with Freund's adjuvant by subcutaneous administration. One week after the fourth vaccine dose, mucosal and serum antibody responses were examined by ELISA using microtiter plates coated with 0.5 µg of native *H. pylori* urease. Serum samples were diluted 1:100 and assayed for urease-specific IgA and IgG. Fresh fecal pellets, extracted with a protease inhibitor buffer (PBS containing 5% non-fat dry milk, 0.2 µM AEBSF, 1 µg aprotinin per ml, and 10 µM leupeptin), were examined for fecal anti-urease IgA antibody. In some experiments, fecal antibody values were normalized for total IgA content determined by ELISA, with urease-specific fecal IgA expressed in A₄₀₅ units/mg total IgA in each sample. Saliva samples were collected after stimulation with pilocarpine under ketamine anesthesia, and tested for urease-specific IgA at a dilution of 1:5.

No significant differences between antibody responses of mice immunized orally with or without NaHCO₃ were detected, and the data were pooled for analysis. Mice given subcutaneous antigen developed urease-specific serum IgG, but serum and fecal IgA responses were elicited only when antigen was delivered by mucosal routes (oral or intragastric).

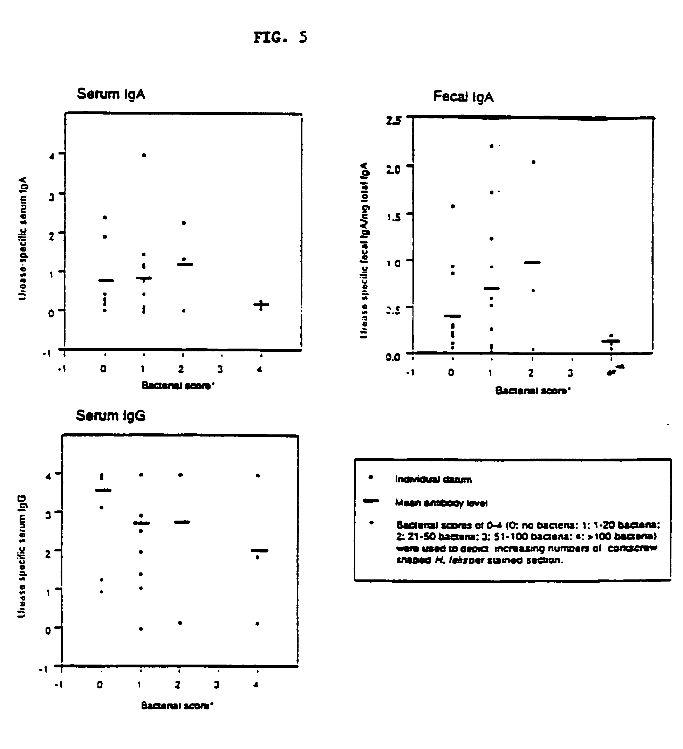

Mice immunized either orally or intragastrically with urease/CT were challenged with 10⁷ *H. felis.* The pre-challenge antibody levels of orally or intragastrically immunized mice were correlated with histologically-determined bacterial scores after *H. felis* challenge (Fig. 5). Although IgA responses varied considerably between individual mice, on the whole, mice with higher serum and fecal IgA levels had reduced bacterial scores. These data indicate a role for IgA in suppression of, and protection from, infection. In contrast, serum IgG antibodies did not correlate with protection. While some protected mice had no detectable IgA antibodies, high levels of anti-urease IgA were not observed in animals that developed 4+ infections after challenge. These results not only support a role for mucosal immune responses in protection, but also suggest that immune mediators other than fecal and serum IgA play a role in eradication of *H*. *felis* infection.

These data show that: i) mucosal immunization is required for protective immunity; ii) the oral route of immunization is as effective, or more effective, than the intragastric route; iii) neutralization of gastric acid with NaHCO₃ is required for effective intragastric (but not oral) immunization; and iv) parenteral immunization does not stimulate mucosal immunity or provide effective protection against challenge.

### Schedule of immunization

Alternative immunization schedules were compared in order to determine the optimal immunization time-table to elicit a protective mucosal immune response. Swiss-Webster mice were immunized with 100 µg recombinant urease in two, three, or four oral administrations, on a schedule shown in Table 2. The mucosal adjuvant CT (10 µg) was co-administered with the recombinant urease. As assessed by qualitative gastric urease assay, mice which received four weekly doses of antigen exhibited the highest levels of protection. Significant protection was also observed in mice given three doses of antigen on days 0, 7, and 21. Secretory IgA antibody responses were highest for mice given a total of four doses of recombinant urease at one week intervals. On the basis of protection ratio and antibody responses, the latter schedule was selected for further evaluation of the therapeutic and prophylactic efficacy of the vaccine.

**Table 2**

| Effect of different schedules of oral immunization on the prophylactic efficacy of urease vaccine | | | |
|---|---|---|---|
| **GROUP** | **VACCINE DOSE/ IMMUNIZATION (µg)^{a}** | **SCHEDULE (DAY OF IMMUNIZATION)** | **% PROTECTED (#/TOTAL)^{b}** |
| 1 | 25 | 0, 7, 14, 21 | 70 (7/10)* |
| 2 | 50 | 0, 14 | 20 (2/10) |
| 3 | 50 | 0, 14 | 30 (3/9) |
| 4 | 33.3 | 0, 7, 21 | 40 (4/10)* |
| 5 | 50, 25, and 25 | 0, 7, 21 | 60 (6/10)* |
| 6 | None | 0, 7, 14, 21 | 0 (0/10) |

| | | | |
|---|---|---|---|
| ^{a} Each group of mice were immunized orally with a total dose of 100 µg recombinant *H. pylori* urease. CT (10 µg) was used at each immunization, suspended in sterile distilled water, as the mucosal adjuvant. Group 6 received CT alone on the same schedule as group 1 and acted as the control. Groups 4 and 5 compared the effect of booster doses administered after two previous weekly immunizations. ^{b} Percent protected (No. protected/No. tested) from a 10⁷ challenge dose of *H. felis* two weeks post-immunization. Mice were sacrificed two weeks after challenge and protection was determined by the qualitative gastric urease assay. * p<0.05, Fisher's exact test, compared to mice given CT alone. | | | |

The effect of different immunization schedules upon anti-urease antibody production was examined in mice. Antibody responses of mice immunized by one of the five different immunization schedules described in Table 2 were examined. Significant protection was observed in mice that received vaccine in four weekly doses or two weekly doses with a boost on day 21. Mice immunized by either of these two immunization schedules also had the highest average immune responses. The serum IgG and salivary IgA levels were highest in mice vaccinated on a schedule of four weekly doses.

### Dose-protection relationship

Graded doses of recombinant urease were orally administered to mice to determine the minimal and optimal doses required for immunization and protection. Recombinant urease doses of 5, 10, 25, 50, and 100 µg were administered to groups of eight mice by the oral route with 10 µg CT in PBS. The antigen was given on a schedule of four weekly doses. As assessed by gastric urease and histologic bacterial score, significant protection of mice against challenge with *H. felis* was observed at all doses, with no significant differences between dose groups (Table 3). A dose response effect was clearly demonstrated in serum and mucosal antibody responses to recombinant urease, with the highest immune response at the 100 µg dose level.

**Table 3**

| Recombinant *H*. *pylori* urease at doses of 5 µg or more, protects mice against challenge with *H. felis* | | | | |
|---|---|---|---|---|
| **VACCINE** | **ADJUVANT** | **ROUTE OF IMMUNIZATION** | **% PROTECTED^{A} (# PROTECTED/TOTAL)** | |
| | | | **UREASE ASSAY** | **HISTOLOGY** |
| none | 10 µg CT | Oral | 0 (0/7) | 0 (0/7) |
| 5 µg rUre | 10 µg CT | Oral | 86 (6/7)* | 71 (5/7)* |
| 10 µg rUre | 10 µg CT | Oral | 88 (7/8)* | 63 (5/8)* |
| 25 µg rUre | 10 µg CT | Oral | 100 (9/9)* | 100 (9/9)* |
| 50 µg rUre | 10 µg CT | Oral | 100 (8/8)* | 88 (7/8)* |
| 100 µg rUre | 10 µg CT | Oral | 100 (7/7)* | 71 (5/7)* |

| | | | | |
|---|---|---|---|---|
| ^{A} Percent protected (number mice with 0-20 bacteria per section/number tested) as determined by examination of silver-stained stomach sections. * Comparison to sham-immunized controls, Fisher's exact test, p≤0.02. | | | | |

The effect of recombinant urease dosage upon the anti-urease antibody response was examined in mice. Swiss-Webster mice were immunized orally with graded doses (0, 5, 10, 25, 50, or 100 µg) of recombinant urease plus 10 µg CT as a mucosal adjuvant. IgA antibody responses in serum, feces, and saliva increased with the amount of recombinant urease administered. A 100 µg dose of recombinant urease produced the highest antibody levels.

Swiss-Webster mice were orally immunized with graded doses (0, 5, 25, or 100 µg) of recombinant urease, with 25 µg enterotoxigenic *E. coli* heat-labile toxin (LT) as a mucosal adjuvant. One group of mice received 25 µg recombinant urease and 10 µg CT as a mucosal adjuvant for comparison. The mice were immunized orally 4 times every 7 days. Serum, feces, and saliva were collected 10 to 13 days after the last immunization and urease-specific antibody levels were determined by ELISA. Mice immunized with recombinant urease and LT developed serum and secretory antibodies against urease, with a clear dose-response effect. Strong salivary IgA antibody responses were observed in these animals.

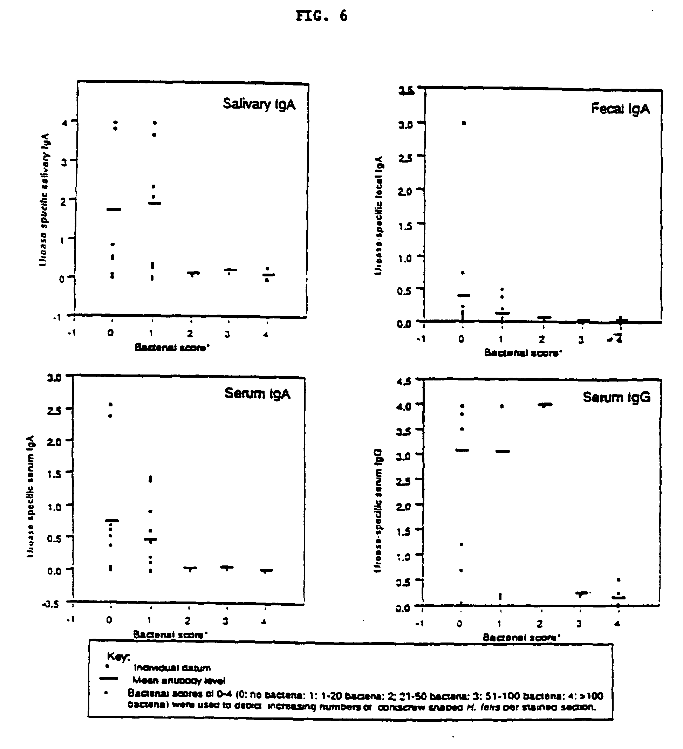

Mice immunized with urease and LT as described above were challenged with 10⁷ *H. felis* (see Fig. 4). The pre-challenge antibody responses of the urease/LT immunized mice were correlated with histologically-determined bacterial scores (Fig. 6). Fully protected animals (0 bacterial score) and those with low-grade infections (1+ bacterial score) had higher levels of antibodies in all compartments than animals with more severe infections. A few protected mice had no detectable immune response, suggesting that immune mediators other than IgA antibodies play a role in eradication of *H. felis* infection.

The ability of high doses of recombinant urease to elicit a mucosal immune response was examined by administering intragastrically 1 µg, 200 µg, or 5 mg without adjuvant. One group of mice were intragastrically immunized with 200 µg urease plus 10 µg CT as a control. Blood, feces, and saliva were collected 5 to 8 days after the last immunization. Animals were then challenged with 10⁷ *H. felis* 10 days after the last immunization and sacrificed 14 days after challenge. *H. felis* infection was detected by urease activity in stomach tissue.

High doses of recombinant urease elicited urease-specific serum IgG, but elicited comparatively low levels of mucosal antibodies as detected by ELISA. Animals which exhibited a urease-specific serum IgG response were fully susceptible to *H. felis* challenge, indicating that serum IgG does not play a role in protection.

In summary, data from the above experiments investigating different administration routes, administration schedules, and mucosal adjuvants demonstrate that, when administered with an effective mucosal adjuvant, oral or intragastric administration of recombinant urease at relatively low doses elicits secretory IgA antibody and serum IgA and IgG responses. Secretory IgA antibody provides protection, while serum IgG responses do not. When protection is measured by histological bacterial counts, animals with higher IgA antibody titers were fully protected, or had significantly reduced infections, as compared to animals with lower IgA antibody titers. Animals which did not exhibit detectable IgA antibody levels developed severe infections after challenge. Antibody responses were dose-dependent and differed by the schedule of administration of antigen. The highest levels were achieved at antigen doses of 100-200 µg. Administration of four doses of antigen at one week intervals provided the optimal schedule for immunization. An additional administration route, intranasal, was investigated, as follows.

### Intranasal vaccination with recombinant urease

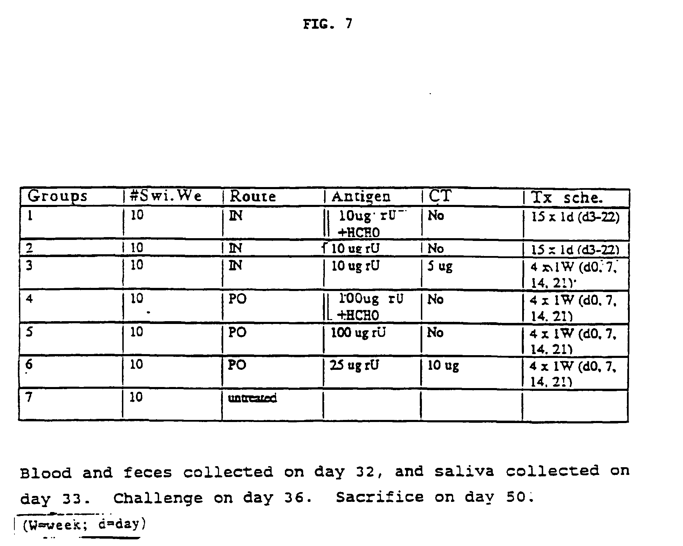

Swiss Webster mice were immunized either orally or intranasally (IN) with recombinant urease or formalin-fixed urease. The amounts of antigen and adjuvant, routes of administration (IN or oral), and immunization schedules are shown in Fig. 7. The formalin-fixed urease (Form-ure) was prepared according to the following protocol:
(1) a vial containing 1 mg recombinant urease is reconstituted with 150 µL of RO/DI water;
(2) 50 µL of formalin (37% formaldehyde), diluted 1:1000 in RO/DI water, is added to the vial (final concentration of urease is 5 mg/ml); and
(3) the vial is incubated at 35°C for 48 hours.

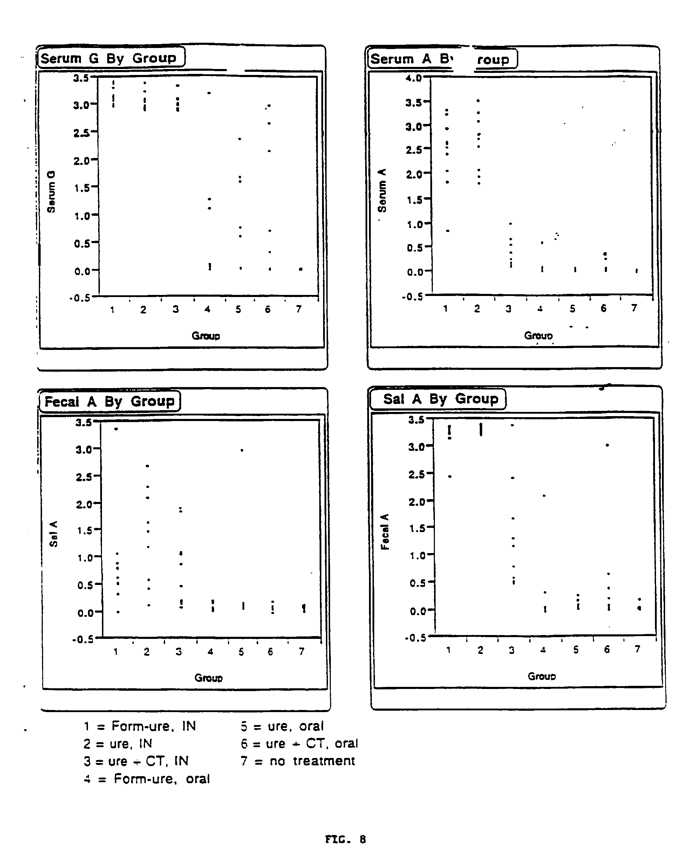

Serum IgG, IgA, fecal IgA and salivary IgA responses in IN + CT group were higher than the oral + CT group, despite higher doses of rUrease and adjuvants that were used for oral immunization (Fig. 7 and Table 4). Protection was measured by the urease test, as well as by determining bacterial scores on stomach tissues of sacrificed animals. One hundred percent protection was found in the IN group, as compared to 80% in the oral group, when protection was assayed by urease test. Seven of eight of the orally immunized mice were positive for bacteria on their stomach tissues, whereas only 1/10 of the mice in the IN group were positive for bacteria (see groups 3 and 6 in Table 4 and Fig. 8).

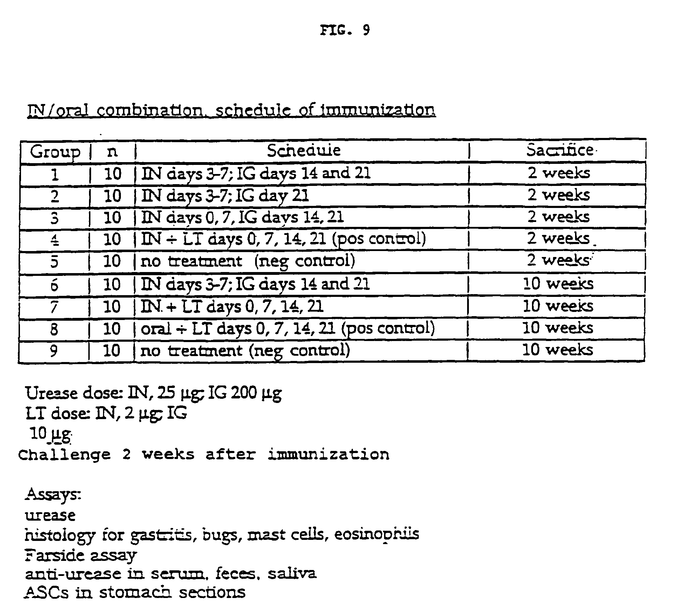

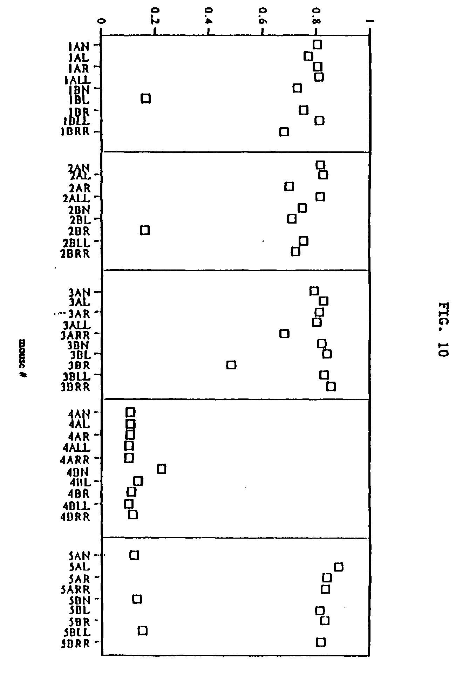

In a second experiment, mice were immunized either intragastrically (IG) or intranasally with rUrease co-administered with LT as mucosal adjuvant (see Fig. 9 for details of the experiment and the immunization schedule). In this experiment, serum IgG, serum IgA, and salivary IgA responses in the IN + LT group were higher than in the IG + LT group, despite higher doses of antigen and adjuvant that were used for IG immunization (Fig. 10 and Table 5, groups 4 and 8). Mice in the IN + LT group were fully protected against *H. felis* challenge (10/10 compared with 3/9 untreated group), as determined by urease test (see groups 4 and 5 of Table 5).

**Table 4**

| Intranasal immunization with recombinant urease and cholera toxin | | | | | | | | | |
|---|---|---|---|---|---|---|---|---|---|
| | Urease | | | | | | four hours | | |
| Mouse # | treatment | route/adjuvant | serum IgG | serum IgA | fecal IgA | salivary IgA | urease | Bact. | Path. |
| 1A1 | 10 µg/HCHO | IN/none | 3.398 | 3.318 | 3.377 | 3.339 | + | | |
| 1A2 | | | 3.119 | 3.218 | 0.786 | 3.249 | + | | |
| 1A3 | | | 2.967 | 2.535 | 0.893 | 3.153 | | | |
| 1A4 | | | 3.064 | 2.390 | 1.065 | 3.311 | + | | |
| 1A5 | | | 3.009 | 2.647 | 0.617 | 3.244 | + | | |
| 1B1 | | | 2.994 | 2.068 | 0.497 | 3.329 | - | | |
| 1B2 | | | 3.099 | 2.611 | -0.010 | 3.235 | + | | |
| 1B3 | | | 3.306 | 1.823 | 0.323 | 3.295 | + | | |
| 1B4 | | | 3.424 | 2.931 | 0.522 | 3.372 | + | | |
| 1B5 | | | 3.175 | 0.832 | 0.803 | 2.439 | + | | |
| | | | | | | | | | |
| 2A1 | 10 µg | IN/none | 3.021 | 2.562 | 2.090 | 3.204 | + | | |
| 2A2 | | | 2.971 | 2.806 | 1.475 | 3.304 | + | | |
| 2A3 | | | 2.894 | 1.943 | 0.584 | 3.288 | + | | |
| 2A4 | | | 2.918 | 2.804 | 2.291 | 3.363 | + | | |
| 2A5 | | | 2.926 | 3.264 | 0.118 | 3.418 | + | | |
| 2B1 | | | 3.220 | 3.505 | 1.634 | 3.415 | + | | |
| 2B2 | | | 3.383 | 2.708 | 0.410 | 3.336 | + | | |
| 2B3 | | | 3.092 | 1.814 | 0.411 | 3.253 | + | | |
| 2B4 | | | 3.033 | 3.090 | 2.672 | 3.266 | + | | |
| 2B5 | | | 3.055 | 2.076 | 1.193 | 3.263 | + | | |
| | | | | | | | | | |
| 3A1 | 10 µg | IN/CT | 2.969 | 0.405 | 0.192 | 1.670 | - | 0 | 2 |
| 3A2 | | | 2.871 | 0.097 | 0.154 | 0.526 | - | 0 | 2 |
| 3A3 | | | 2.984 | 0.677 | 0.081 | 0.473 | - | | |
| 3A4 | | | 3.092 | 0.563 | 1.824 | 3.398 | | | |
| 3A5 | | | 3.335 | 0.256 | 1.052 | 0.587 | - | 1 | 2 |
| 3B1 | | | 3.013 | 0.148 | 0.142 | 0.586 | - | 0 | 2 |
| 382 | | | 3.068 | 0.388 | 0.867 | 2.410 | - | 0 | 2 |
| 3B3 | | | 3.008 | 0.255 | 0.468 | 0.778 | - | 0 | 2 |
| 3B4 | | | 2.908 | 0.985 | 1.086 | 1.172 | - | 0 | 2 |
| 385 | | | 2.879 | 0.186 | 1.904 | 1.317 | - | 0 | 2 |
| | | | | | | | | | |
| 4A1 | 100 µg/HCHO | oral/none | -0.006 | 0.024 | 0.021 | 0.009 | + | | |
| 4A2 | | | 0.050 | 0.097 | 0.079 | 0.041 | + | | |
| 4A3 | | | 0.001 | 0.097 | 0.056 | -0.019 | + | | |
| 4A4 | | | 3.195 | 0.598 | 0.190 | 2.097 | + | 0 | 3 |
| 4A5 | | | 1.271 | 0.027 | 0.034 | 0.010 | + | | |
| 4B1 | | | 0.096 | 0.045 | 0.165 | 0.309 | + | | |
| 4B2 | | | 0.021 | 0.033 | 0.043 | 0.059 | + | | |
| 4B3 | | | 1.109 | 0.055 | 0.080 | 0.019 | + | | |
| 4B4 | | | 0.006 | 0.022 | 0.083 | 0.017 | + | | |
| 4B5 | | | 0.013 | 0.027 | 0.039 | 0.010 | + | | |
| 5A1 | 100 µg/-- | oral/none | 0.032 | 0.042 | 0.151 | 0.030 | + | | |
| 5A2 | | | 0.756 | 0.077 | 2.957 | 0.156 | + | | |
| 5A3 | | | 2.362 | 0.075 | 0.104 | 0.262 | + | | |
| 5A4 | | | 0.012 | 0.034 | 0.146 | 0.065 | + | | |
| 5A5 | | | 0.011 | 0.035 | 0.163 | 0.013 | + | | |
| 5B1 | | | 0.012 | 0.042 | 0.089 | 0.017 | + | | |
| 5B2 | | | 0.017 | 0.017 | 0.076 | 0.049 | + | | |
| 5B3 | | | 1.583 | 0.058 | 0.058 | 0.182 | +/- | | |
| 5B4 | | | 0.602 | 0.030 | 0.093 | 0.093 | + | | |
| 5B5 | | | 1.672 | 0.059 | 0.060 | 0.093 | + | | |
| | | | | | | | | | |
| 6A1 | 25 µg/-- | oral/CT | 0.698 | 0.267 | 0.076 | 0.387 | - | 1 | 2 |
| 6A2 | | | 2.139 | 0.065 | 0.089 | 0.206 | - | 1 | 2 |
| 6A3 | | | 0.006 | 0.013 | 0.098 | 0.011 | - | 3 | 2 |
| 6A4 | | | 2.957 | 0.354 | 0.185 | 2.999 | - | 1 | 2 |
| 6A5 | | | 0.011 | 0.019 | 0.042 | 0.026 | - | 0 | 2 |
| 6B1 | | | 0.000 | 0.022 | 0.041 | 0.005 | - | 1 | 2 |
| 6B2 | | | 0.009 | 0.002 | 0.067 | 0.069 | - | 2 | 2 |
| 6B3 | | | 0.320 | 0.022 | 0.041 | 0.048 | + | 4 | 2 |
| 6B4 | | | 0.010 | 0.009 | -0.021 | 0.023 | + | 4 | 2 |
| 6B5 | | | 2.633 | 0.090 | 0.047 | 0.647 | - | 1 | 2 |
| | | | | | | | | | |
| 7A1 | none | | 0.011 | 0.018 | 0.074 | 0.019 | + | 4 | 1 |
| 7A2 | | | 0.007 | 0.025 | 0.042 | -0.004 | + | 4 | 2 |
| 7A3 | | | 0.000 | 0.019 | 0.082 | 0.018 | + | 4 | 2 |
| 7A4 | | | 0.004 | 0.028 | 0.024 | -0.002 | + | 4 | 2 |
| 7A5 | | | 0.011 | 0.005 | 0.041 | 0.029 | X | | |
| 7B1 | | | 0.019 | 0.004 | 0.089 | -0.005 | + | 4 | 1 |
| 7B2 | | | 0.006 | -0.003 | -0.009 | 0.008 | + | 4 | 2 |
| 7B3 | | | 0.006 | 0.024 | 0.066 | 0.167 | + | 4 | 2 |
| 7B4 | | | 0.015 | 0.018 | 0.063 | 0.018 | + | 4 | 1 |
| 7B5 | | | 0.007 | 0.023 | 0.111 | 0.011 | + | | |
| | | | | | | | | | |
| plate 1 | controls | | | | | | | | |
| + | | | 2.265 | 3.280 | 1.651 | 3.315 | | | |
| - | | | -0.010 | 0.040 | 0.065 | 0.056 | | | |
| | | | | | | | | | |
| plate 2 | controls | | | | | | | | |
| + | | | 1.969 | 3.289 | 1.700 | 3.298 | | | |
| - | | | -0.001 | 0.034 | -0.004 | 0.019 | | | |

**Table 5**

| Intranasal immunization with recombinant urease and LT | | | | | |
|---|---|---|---|---|---|
| | Urease | | | | 2 week sacrifice |
| Mouse # | Treatment | serum IgG | serum IgA | salivary IgA | Absorbance₅₅₀ |
| 1AN | 25 µg 5x IN | 3.530 | 0.350 | 0.340 | 0.802 |
| 1AL | 200 µg 2x IG | 3.263 | 0.154 | 1.544 | 0.768 |
| 1AR | | 3.231 | 0.630 | 0.204 | 0.805 |
| 1ALL | | 3.233 | 0.318 | 0.251 | 0.806 |
| 1BN | | 3.344 | 0.401 | 0.112 | 0.726 |
| 1BL | | 3.322 | 0.724 | 2.015 | 0.168 |
| 1BR | | 3.424 | 0.225 | 0.426 | 0.748 |
| 1BLL | | 3.285 | 0.591 | 0.719 | 0.807 |
| 1BRR | | 3.262 | 0.141 | 0.202 | 0.675 |
| | | | | | |
| 2AN | 25 µg 5x IN | 3.164 | 0.054 | 0.014 | 0.814 |
| 2AL | 200 µg 1x IG | 3.232 | 0.231 | 0.747 | 0.823 |
| 2AR | | 3.236 | 0.981 | 0.412 | 0.695 |
| 2ALL | | 3.303 | 0.122 | 0.832 | 0.813 |
| 2BN | | 3.357 | 0.690 | 0.993 | 0.747 |
| 2BL | | 3.264 | 1.002 | 0.840 | 0.704 |
| 2BR | | 3.284 | 1.377 | .0934 | 0.161 |
| 2BLL | | 3.241 | .0458 | 0.191 | 0.748 |
| 2BRR | | 3.280 | 0.129 | 0.134 | 0.720 |
| | | | | | |
| 3AN | 25 µg 2x IN | 0.165 | 0.031 | 0.001 | 0.791 |
| 3AL | 200 µg 2x IG | 3.364 | 0.332 | 0.356 | 0.824 |
| 3AR | | 1.731 | 0.031 | -0.003 | 0.810 |
| 3ALL | | 3.321 | 0.450 | 0.541 | 0.798 |
| 3ARR | | 3.358 | 0.158 | 0.001 | 0.675 |
| 3BN | | 3.199 | 0.276 | 0.212 | 0.820 |
| 3BL | | 3.174 | 2.463 | 2.838 | 0.839 |
| 3BR | | 3.274 | 0.607 | 1.517 | 0.481 |
| 3BLL | | 3.284 | 1.279 | 2.593 | 0.829 |
| 3BRR | | 3.254 | 0.427 | 1.684 | 0.852 |
| | | | | | |
| 4AN | 25 µg 4x IN | 3.375 | 0.118 | 0.212 | 0.111 |
| 4AL | with 2 µg LT | 3.482 | 0.346 | 0.546 | 0.107 |
| 4AR | | 3.330 | 3.237 | 3.302 | 0.109 |
| 4ALL | | 3.343 | 3.067 | 3.229 | 0.103 |
| 4ARR | | 3.220 | 1.625 | 2.156 | 0.106 |
| 4BN | | 3.287 | 1.263 | 2.943 | 0.224 |
| 4BL | | 3.305 | 0.496 | 2.029 | 0.139 |
| 4BR | | 3.283 | 0.809 | 2.307 | 0.114 |
| 4BLL | | 3.313 | 1.019 | 3.346 | 0.106 |
| 4BRR | | 3.474 | 0.674 | 1.683 | 0.119 |
| 5AN | none | 0.075 | 0.023 | 0.001 | 0.126 |
| 5AL | | 0.005 | 0.016 | -0.002 | 0.881 |
| 5AR | | 0.011 | 0.008 | -0.001 | 0.839 |
| 5ARR | | 0.005 | 0.007 | 0.000 | 0.834 |
| 5BN | | 0.033 | 0.016 | 0.001 | 0.134 |
| 5BL | | 0.013 | 0.009 | 0.004 | 0.814 |
| 5BR | | 0.025 | 0.014 | 0.007 | 0.833 |
| 5BLL | | 0.028 | 0.019 | 0.008 | 0.155 |
| 5BRR | | 0.095 | 0.012 | 0.003 | 0.818 |
| | | | | | |
| 6AN | 25 µg 5x IN | 3.353 | 0.680 | 0.498 | |
| 6AL | 200 µg 1x IG | 3.470 | 0.248 | 0.100 | |
| 6AR | | 3.387 | 1.236 | 0.741 | |
| 6ALL | | 3.362 | 0.409 | 0.322 | |
| 6ARR | | 3.244 | 1.036 | 1.056 | |
| 6BN | | 3.211 | 2.749 | 2.014 | |
| 6BL | | 3.286 | 0.360 | 0.328 | |
| 6BR | | 3.269 | 0.749 | 1.882 | |
| 6BLL | | 3.347 | 0.275 | 0.030 | |
| 6BRR | | 3.321 | 0.311 | 0.989 | |
| | | | | | |
| 7AN | 25 µg 4x IN | 3.415 | 0.756 | 2.584 | |
| 7AL | with 2 µg LT | 3.428 | 3.080 | 3.287 | |
| 7AR | | 3.249 | 2.054 | 3.188 | |
| 7ALL | | 3.269 | 1.010 | 3.240 | |
| 7ARR | | 3.255 | 0.713 | 3.243 | |
| 7BN | | 3.310 | 3.081 | 3.274 | |
| 7BL | | 3.439 | 2.298 | 3.222 | |
| 7BR | | 3.359 | 1.026 | 1.757 | |
| 7BLL | | 3.370 | 1.441 | 3.025 | |
| 7BRR | | 3.330 | 0.689 | 1.169 | |
| | | | | | |
| 8AN | 200 µg 4x IG | 0.281 | 0.031 | 0.000 | |
| 8AL | with 10 µg LT | 3.234 | 0.925 | 1.872 | |
| 8AR | | 3.285 | 0.827 | 2.751 | |
| 8ALL | | 3.313 | 0.547 | 0.395 | |
| 8ARR | | 3.363 | 0.382 | 1.144 | |
| 8BN | | 3.407 | 1.189 | 1.880 | |
| BBR | | 3.315 | 1.608 | 2.536 | |
| 8BLL | | 3.271 | 0.438 | 0.599 | |
| | | | | | |
| 9AN | none | 0.011 | 0.003 | 0.000 | |
| 9AL | | 0.017 | 0.005 | 0.001 | |
| 9AR | | 0.007 | 0.002 | 0.001 | |
| 9ALL | | 0.011 | 0.013 | 0.000 | |
| 9ARR | | 0.006 | 0.019 | -0.002 | |
| 9BN | | 0.017 | 0.010 | -0.001 | |
| 9BL | | 0.020 | 0.008 | -0.002 | |
| 9BR | | 0.031 | 0.012 | -0.002 | |
| 9BLL | | 0.005 | 0.016 | 0.005 | |
| 9BRR | | 0.019 | 0.011 | 0.000 | |

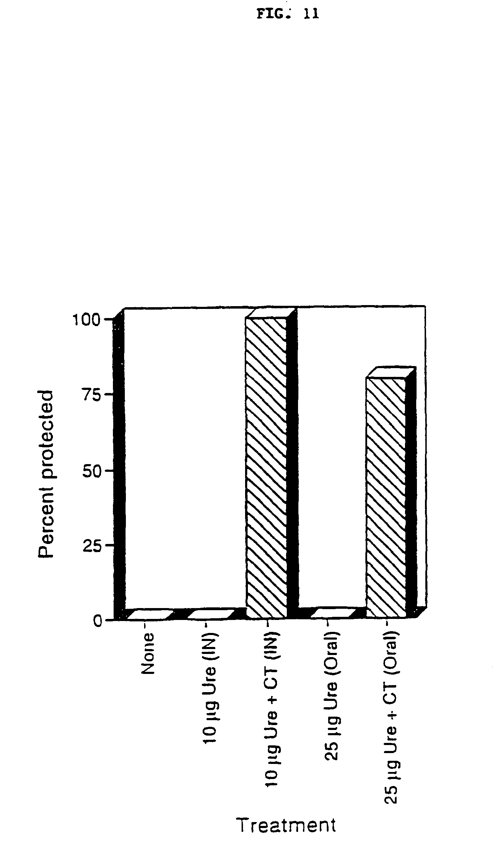

Further support for the efficacy of intranasal immunization is shown in Fig. 11. Briefly, this experiment showed that rUrease (10 µg), administered by the intranasal route with CT (5 µg), is at least as effective as rUrease (25 µg) given by the oral route with CT (10 µg) in preventing infection with *H. felis*.

### Selection of a mucosal adjuvant

*E. coli* heat-labile toxin (LT) is a multisubunit toxin which is closely related, both biochemically and immunologically, to CT. Because the toxicity of LT is lower than CT, LT is a practical mucosal adjuvant for use in humans (Walker and Clements, *Vaccine Res.* 2:1-10, 1993).

Mice were orally immunized with 5 µg, 25 µg, or 100 µg recombinant urease and 25 µg recombinant LT (Swiss Serum Vaccine Institute, Berne, Switzerland) for a total of four weekly doses. Control mice received LT only or 25 µg urease vaccine with CT. Mice were challenged two weeks after the fourth dose and necropsied two weeks after challenge.

Gastric urease assays indicated significant protection or suppression of the infection in immunized animals at all vaccine doses (Table 6). Histological assessment confirmed significant reductions in bacterial scores in mice which received ≤ 5 µg urease and LT. Protection was directly correlated with dose as determined by both gastric urease assay and histological examination, with the highest protection conferred by 100 µg of recombinant urease co-administered with LT. Antibody determinations confirmed a dose-response relationship, with highest mucosal immune responses at the 100 µg dose. When recombinant urease was administered at equivalent doses (25 µg), LT was superior to CT as a mucosal adjuvant. These data indicate that while CT enhances the mucosal immune response to orally administered recombinant urease, LT is a better mucosal adjuvant and is thus the preferred over CT for coadministration with recombinant urease.

**Table 6**

| *E. coli* heat-labile enterotoxin (LT) as a mucosal adjuvant for immunization with recombinant *H. pylori* urease | | | |
|---|---|---|---|
| **VACCINE** | **ADJUVANT** | **% PROTECTED (# PROTECTED/TOTAL)^{a}** | |
| | | **UREASE ASSAY** | **HISTOLOGY** |
| None | 25 µg LT | 0 (0/9) | 0 (0/9) |
| 25 µg rUre | 10 µg CT | 70 (7/10)* | 60 (6/10)* |
| 5 µg rUre | 25 µg LT | 787 (7/9)* | 56 (5/9)* |
| 25 µg rUre | 25 µg LT | 90 (9/10)* | 80 (8/10)* |
| 100 µg rUre | 25 µg LT | 100 (8/8)* | 100 (8/8)* |

| | | | |
|---|---|---|---|
| ^{a} Percent protected (number of mice with negative urease assay or bacterial score 0-1+/number tested) determined by bacterial scores of silver-stained stomach sections. * Comparison to sham-immunized controls, Fisher's exact test, p<0.03. | | | |

To determine the adjuvant activity of lower doses of LT, mice were orally immunized with 25 µg of recombinant urease administered with 1, 5, 10, or 25 µg of LT. Similar protection ratios and antibody responses were observed at all LT doses.

Several studies were performed to assess adjuvants other than CT and LT, and to determine whether the requirement for a mucosal adjuvant could be eliminated by administration of antigen alone at a high dose. Cholera toxin B subunit (CTB) was compared with CT as a mucosal adjuvant for urease immunization. No protective effect was observed when 200 µg urease was co-administered intragastrically with 100 µg CTB (Calbiochem, La Jolla, CA) in 0.24 M sodium bicarbonate, whereas the same amount of urease with 10 µg CT gave 100% protection, as determined by gastric urease activity.

An orally-active semi-synthetic analogue of muramyl dipeptide, GMDP, (N-acetylglucosaminyl-(b1-4)-N-acetyl-muramyl-L-alanyl-D-isoglutamine), was co-administered with 25 µg urease at doses of 2, 20, and 200 µg. GMDP co-administered with recombinant urease failed to protect mice against *H. felis* challenge.

Large doses (200 µg, 1 mg, or 5 mg) of recombinant urease with or without CT were intragastrically administered to mice once a week for four weeks. The intragastric route was required because volumes for high antigen doses exceeded those that could be given orally in a reproducible fashion. NaHCO₃ was co-administered to neutralize gastric acid. A total of four doses of antigen were administered at ten day intervals. Blood, feces, and saliva were collected five to eight days after the last immunization. Animals were challenged with 1 x 10⁷ *H*. *felis,* and infection was determined by urease activity in stomach tissue.

In the absence of CT, high antigen doses did not confer protection against *H. felis* challenge, whereas controls given 200 µg of recombinant urease with CT were significantly protected (Table 7). Urease-specific serum IgG was induced at the high recombinant urease doses without adjuvant, but serum, fecal, and salivary IgA responses were absent or minimal. Histological examination of coded specimens from animals given 5 mg doses of urease revealed no differences in bacterial scores or leukocytic infiltrates, compared with sham-immunized animals.

**Table 7 A mucosal adjuvant facilitates protection by recombinant urease**

| **Vaccine** | **Adjuvant** | **% protected (# protected/ tested)** | **Mean antibody levels (±SD) before challenge^{a}** | | | |
|---|---|---|---|---|---|---|
| | | | **Serum IgG** | **Serum IgG** | **Fecal IgA** | **salivary IgA** |
| none | PBS | 0 (0/5) | 0.01 | 0.03 | 0.01 | 0.02 |
| | | | (±0.00) | (±0.07) | (±0.0 2) | (±0.03) |
| 200 µg rUre | 10 µg CT | 88 (7/8)* | >2.97 | >1.02 | 0.16 | 0.59 |
| | | | (±1.84) | (±1.41) | (±0.1 4) | (±0.73) |
| 200 µg rUre | none | 0 (0/9) | >2.06 | 0.07 | 0.02 | 0.06 |
| | | | (±1.98) | (±0.11) | (±0.0 2) | (±0.13) |
| 1 mg rUre | none | 0 (0/9) | >3.28 | 0.12 | 0.02 | 0.18 |
| | | | (±1.48) | (±0.17) | (±0.0 3) | (±0.40) |
| 5 mg rUre | none | 0 (0/9) | >2.68 | >0.22 | 0.02 | 0.08 |
| | | | (±1.98) | (±0.17) | (±0.0 2) | (±0.11) |

| | | | | | | |
|---|---|---|---|---|---|---|
| ^{a} Mean (±SD) urease-specific serum IgG or IgA expressed as A₄₀₅ units of serum (diluted 1:100), fecal extract (diluted 1:20), or saliva (diluted 1:5). Means are shown as > when individual values of 4.0 (the maximum A₄₀₅ reading) were included in calculating the mean values. * Comparison to sham-immunized controls, Fisher's exact test, p=0.005. | | | | | | |

### Therapeutic immunization of mice with H. felis gastritis

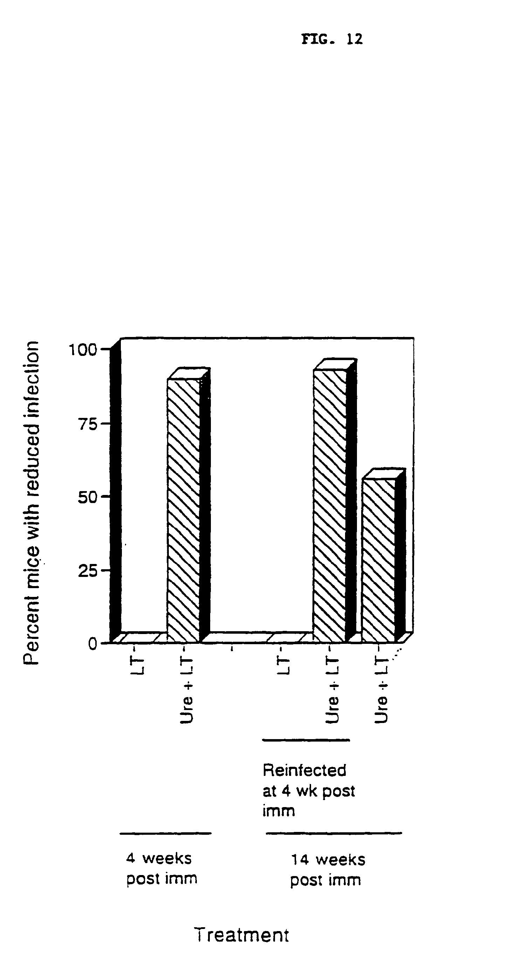

The efficacy of the recombinant urease vaccine in treating *Helicobacter* infection in mice was evaluated by intragastrically infecting Balb/c mice with 10⁷ *H. felis,* and, four weeks after infection, orally administering to the infected mice four weekly doses of 100 µg recombinant urease with 10 µg LT. Control mice received LT only (Fig. 12).

Ten of the mice in each group were necropsied four weeks after the final immunization for examination of the degree of *Helicobacter* infection by quantitative gastric urease. Nine out of ten Balb/c mice were free from infection, as measured by quantitative urease assay, whereas all controls were infected.

At four weeks, twelve animals receiving LT and 40 animals receiving urease + LT were reinfected with *H. felis.* Ten weeks after the challenge, the animals were sacrificed to determine the extent of infection by quantitative urease assay. Of nine animals which were given urease + LT, but not reinfected, 5 were still clear of infection (57%), as determined by gastric urease activity. All twelve LT-treated animals which were rechallenged were infected. Thirty-seven of the 40 mice (93%) which were given urease + LT, and then rechallenged with *H. felis,* were protected as determined by reduced gastric urease activity. This experiment shows that urease vaccination not only eradicates an existing *Helicobacter* infection, but also protects the host against reinfection.

Five of fourteen (36%) immunized Swiss-Webster mice were cured or had reduced infections, whereas all control animals were infected, although the differences in infection ratios between groups was not significant (p=0.26, Fisher's exact test, two-tailed). Infection in Swiss-Webster mice was more severe than in Balb/c mice, as measured by higher mean gastric urease activity in unimmunized animals (p < 0.0001, one-way ANOVA), possibly explaining the lower cure rates in Swiss-Webster versus Balb/c mice. Differences in susceptibility of mouse strains to *H. felis* has been noted (Sakagami et al., Am. J. Gastroenterol 89:1345, 1994).

By histologic assessment, all unimmunized Balb/c mice had 4+ infections (> 100 bacteria/section), whereas reduced bacterial scores were seen in 43% of immunized mice at four weeks. At ten weeks, four of six had reduced urease activity, although only one of six had a reduced bacterial score.

### The role of antibodies in Helicobacter therapy

The role of anti-urease antibodies in *Helicobacter* therapy, *i*.*e*., the clearance of *H. felis* from infected mice, was examined by first infecting Balb/c mice with 10⁷ *H. felis.* Four weeks after infection, the mice were orally immunized with 200 µg recombinant urease plus 10 µg CT. Control mice were given 10 µg CT only. Antigen was administered 4 times at one week intervals. Animals were sacrificed 4 and 10 weeks after the last immunization, and serum and fecal samples were collected for ELISA.

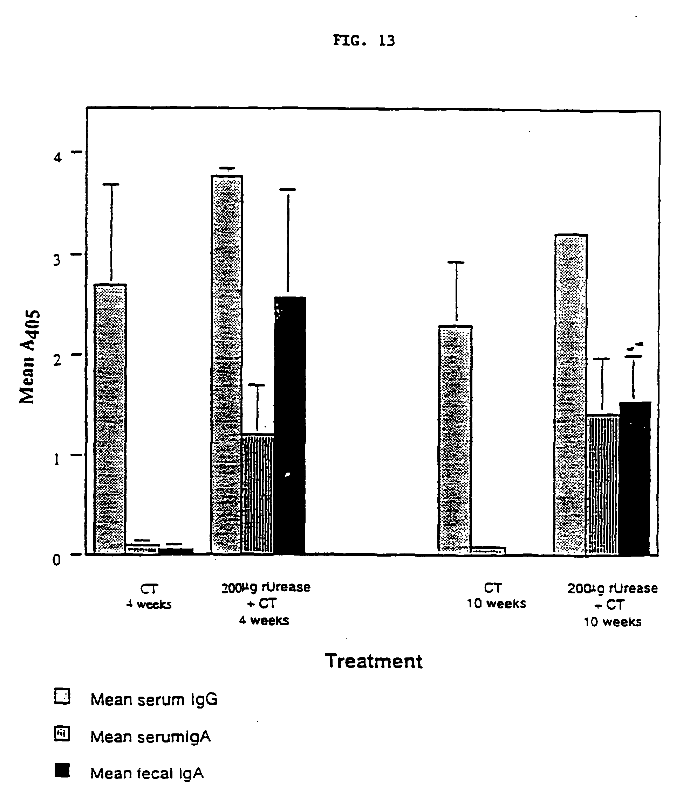

Mice infected with *H. felis* produced serum anti-urease IgG antibodies, but no secretory anti-urease IgA response was detected. However, infected animals immunized with urease/CT exhibited high secretory anti-urease IgA antibody responses (Fig. 13). There was no significant difference in urease specific mucosal IgA levels between immunized mice that remained infected and those with reduced bacterial scores.

These data indicate that *H. felis* infection does not elicit a secretory anti-urease response. Thus, suppression of the IgA antibody response may play a role in the ability of *H. felis* to evade clearance by the immune system. In contrast, immunization of *H. felis-*infected mice with urease and a mucosal adjuvant resulted in strong mucosal anti-urease responses, which correlated with clearance of the *H. felis* infection.

### Correlation of protection against Helicobacter infection with gastric immune responses

Several of the experiments using the mouse infection model showed that some animals rendered resistant to infection by recombinant urease vaccine lacked detectable antibody responses, or had low antibody levels, in serum, saliva, or feces. Thus, the immune response was measured in the gastric mucosa itself, in order to determine whether measurement of an immune response could be more precisely correlated with protection.

Immune responses in gastric mucosa were assessed by detecting IgA antibodies and IgA-positive antibody secreting cells in intestinal and gastric murine tissue by immunohistochemistry. Portions of the stomach containing pylorus-proximal duodenum, antrum, corpus, and cardia were mounted in OCT compound, flash-frozen, and cryosectioned. Sections (7 µm thick) were fixed in cold acetone, and IgA-positive cells were identified by staining with biotinylated monoclonal anti-IgA, followed by avidin conjugated to biotinylated glucose oxidase (ABC-GO, Vector Laboratories, Burlingame, CA), and counterstained with methyl green. Urease-specific antibody secreting cells (ASC) were identified by sequentially incubating sections with recombinant urease, rabbit anti-urease, biotinylated donkey anti-rabbit Ig (Amersham, Arlington Heights, IL), ABC-GO, TNBT, and methyl green. Control sections were incubated without urease or urease plus rabbit anti-urease to determine reactivity with the donkey secondary reagent and background endogenous glucose oxidase activity. Cryosections of cell pellets from a hybridoma which produces a monoclonal IgA antibody against *H. felis* ureB (MAB71) and an irrelevant IgA monoclonal (HNK20) against F glycoprotein of respiratory syncytial virus served as positive and negative controls, respectively.

Swiss-Webster mice were immunized orally with four weekly doses of 100 µg recombinant urease plus adjuvant (CT). Control mice received adjuvant only. Groups of three mice each were necropsied at 3, 7, 14, or 21 days after the last immunization. Peyer's patches were removed from the intestines and lamina propria lymphocytes (LPL) were isolated by separation on a 40-70% Percoll gradient. IgA-positive B cells were detected by ELISPOT assays in 96-well filter plates coated with 1 µg/well recombinant urease and blocked with bovine serum albumin. Ten-fold serial dilutions of LPL were added to the wells, starting at 1 x 10⁶ cells. IgA-positive ASC were detected with a biotinylated anti-mouse IgA reagent followed by streptavidin-alkaline phosphatase, and positive cells were counted by microscopy.

Anti-urease IgA-positive ASC were found by ELISPOT in intestinal lamina propria as early as three days after the last immunization, peaked at seven days, and diminished thereafter (Table 8). Urease-specific ASC represented ∼10% of the total IgA-positive cells observed on immunohistochemistry. Two-color immunofluorescence microscopy confirmed that urease-specific ASC were also IgA-positive. These observations confirm that an intense anti-urease IgA response occurs at the level of the intestinal mucosa after oral antigenic stimulation. The chronology and kinetics of this response are similar to those described for other oral vaccines (Czerkinsky et al., Infect. Immun. 59:996-1001, 1991; McGhee and Kyono, Infect. Agents Hum. Dis. 2:55-73, 1993).

**Table 8**

| Kinetics of induction of anti-urease IgA secreting cells after oral immunization with recombinant urease | | | | |
|---|---|---|---|---|
| **IMMUNIZATION** | **NUMBER OF ASC/10⁶ LAMINA PROPRIA LYMPHOCYTES** | | | |
| | **DAY 3^{a}** | **DAY 7** | **DAY 14** | **DAY 21** |
| recombinant urease + CT^{b} | 17^{c} | 7400 | 10 | 2 |
| PBS + CT | 1 | 200 | 0 | 0 |

| | | | | |
|---|---|---|---|---|
| ^{a} Day after the last oral immunization. ^{b} Cholera toxin. ^{c} Average value from duplicate wells containing intestinal lymphocytes from three separate mice. | | | | |

To determine whether IgA-positive cells are recruited into the gastric mucosa, stomachs of orally immunized mice were examined by immunohistochemistry, as is described above. IgA-positive cells were virtually absent in gastric mucosa of immunized only and control mice, indicating that the stomach is immunologically "silent" until stimulated by *Helicobacter* challenge.

The role of the stomach as an immunological effector organ in challenged, immunized mice was examined. Mice were given four weekly oral doses of 200 µg recombinant urease with 10 µg CT. Control mice received CT only. One week after immunization, the mice were challenged. Mice were necropsied prior to challenge and at 7, 14, 28, 70, and 133 days after challenge.

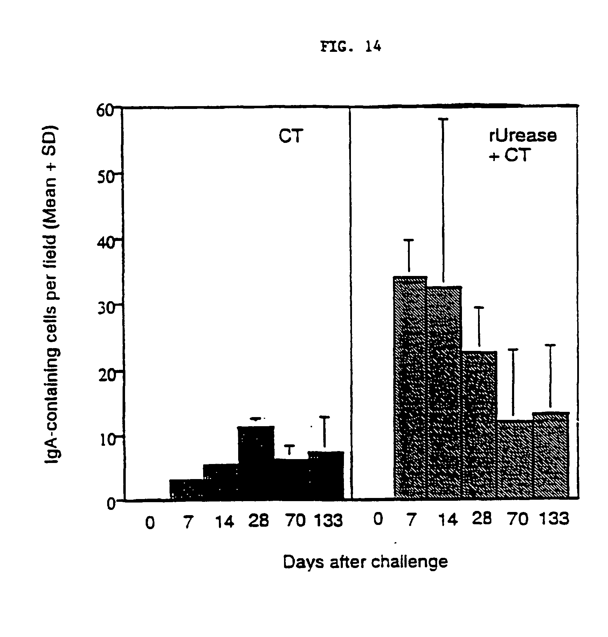

Prior to challenge, no IgA-positive ASC were found. At all time intervals after challenge, IgA-positive ASC were present in large numbers in the gastric mucosae of immunized mice, with a peak at seven days (Fig. 14). The number of IgA-positive ASC greatly exceeded that in unimmunized (CT only) mice, especially 7-28 days after challenge. The anatomical localization of IgA-positive ASC also differed, with immunized mice having cells throughout the mucosa, in the lamina propria, and around the crypts, but rarely under the surface epithelium. Urease-specific and IgA-positive ASC revealed that the majority of urease-specific cells in gastric mucosa were IgA-positive.

These observations indicate that the gastric mucosa of animals primed by prior immunization becomes immunologically activated only after antigenic stimulation by *H. felis*. The resulting tissue response is characterized by rapid, intense, and long-lasting recruitment of IgA-positive B cells, many of which are urease specific. This response is quantitatively greater than in immunologically naive mice after challenge. Moreover, the localization of IgA-positive cells in immunized mice differs from that in immunologically naive mice that are challenged and become persistently infected with *H*. *felis.* The enhanced IgA-positive ASC response in gastric mucosa suggests a basis for the protection conferred by immunization against *H. felis* challenge. These data are concordant with studies of cholera vaccine, in which immunological memory responses triggered within hours after bacterial challenge were sufficient to provide protection (Lycke and Holmgren, Scand. J. Immunol. 25:407-412, 1987).

### Correlation of the gastric immune response and bacterial load

The relationship between the gastric tissue immune response and bacterial infection was defined at the structural level. Swiss-Webster mice were immunized with 4 weekly doses of 200 µg recombinant urease with 10 µg CT. One week after the last immunization, the mice were challenged with 10⁷ *H. felis.* Animals were sacrificed 0, 1, 7, 14, 28, 70, and 133 days after challenge, and *H. felis* colonization was assessed by light and electron microscopy.

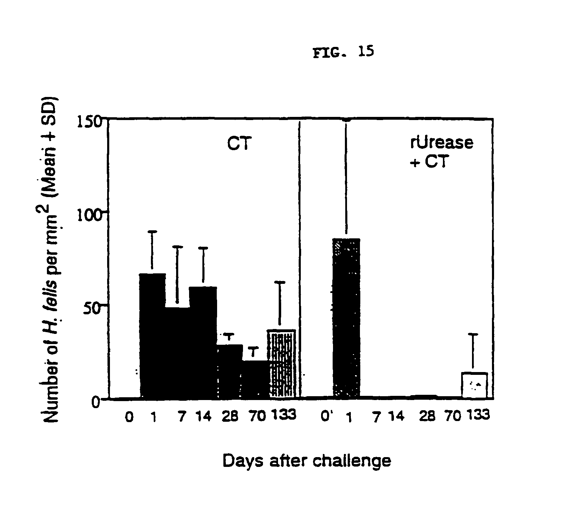

Within 24 hours after challenge, both immunized and unimmunized mice had substantial numbers of *H. felis* within the lumen of gastric pits (Fig. 15). Within seven days after challenge, the bacteria were cleared from the immunized mice, but were still present in high numbers in the gastric pits and the lumens of unimmunized mice. Bacteria were also associated with the apical membrane of mucus-secreting cells of the unimmunized mice. The clearance of bacteria from the gastric mucosa of immunized mice corresponded to the appearance of IgA-positive ASC and anti-urease ASC in the gastric tissue.

These results suggest the following sequence of events: 1) challenge of immunized mice results in transient colonization of gastric epithelium with *H. felis;* 2) unimmunized animals remain infected, while animals immunized with recombinant urease clear the bacteria from the stomach during the first week after challenge; and 3) clearance of infection is associated with the recruitment of IgA-positive urease-specific ASC to gastric mucosa. A similar mechanism may be responsible for the clearance of bacteria from the gastric mucosa of chronically infected animals that are subjected to therapeutic immunization.

### Antigenic conservation of urease among strains of H. pylori

The ability of various antisera to bind multiple clinical isolates of *H. pylori* was tested. The antisera included MPA3, a hyperimmune rabbit serum prepared against purified *H. pylori* urease, and sera and secretions (gastric wick samples and saliva) from mice immunized with recombinant urease. Antibody preparations were tested by immunoblotting for recognition of the homologous *H. pylori* strain (Hp630), ATCC 43504 type strain, and five clinical isolates from ulcer patients at St. Bartholomew's Hospital, London, collected within the last five years.

All antisera recognized the UreA and UreB subunits of all *H. pylori* strains, as well as purified native and recombinant *H. pylori* ureases, native and recombinant *H. felis* urease, and native *H*. *mustelae* urease. In addition, urease-specific IgA antibodies in gastric secretions and saliva of immunized mice reacted with both UreA and UreB of all *H. pylori* strains, as well as heterologous ureases. Immunologic recognition was greater for UreB than for UreA. Sham-immunized mice showed no reactivity with any urease subunits. These results demonstrate that *H. pylori* strains express ureases that are highly conserved at the antigenic level. Thus, antigenic variation among *H*. *pylori* strains is not a significant factor for development of a recombinant urease vaccine.

### Combination methods and compositions for treating Helicobacter infection

Gastroduodenal infections, such as *Helicobacter* infection, may be treated by oral administration of a vaccine antigen (*e*.*g*., *H. pylori* urease) and a mucosal adjuvant, in combination with an antibiotic, an antisecretory agent, a bismuth salt, an antacid, sucralfate, or a combination thereof. Examples of such compounds which may be administered with the vaccine antigen and the adjuvant are: antibiotics, including, *e*.*g*., macrolides, tetracyclines, β-lactams, aminoglycosides, quinolones, penicillins, and derivatives thereof (specific examples of antibiotics that may be used in the invention include, *e*.*g*., amoxicillin, clarithromycin, tetracycline, metronidizole, erythromycin, cefuroxime, and erythromycin); antisecretory agents, including, *e*.*g*., H₂-receptor antagonists (*e*.*g*., cimetidine, ranitidine, famotidine, nizatidine, and roxatidine), proton pump inhibitors (*e*.*g*., omeprazole, lansoprazole, and pantoprazole), prostaglandin analogs (*e*.*g*., misoprostil and enprostil), and anticholinergic agents (*e*.*g*., pirenzepine, telenzepine, carbenoxolone, and proglumide); and bismuth salts, including colloidal bismuth subcitrate, tripotassium dicitrate bismuthate, bismuth subsalicylate, bicitropeptide, and pepto-bismol (see, *e*.*g*., Goodwin et al., Helicobacter pylori, Biology and clinical Practice, CRC Press, Boca Raton, FL, pp 366-395, 1993; Physicians' Desk Reference, 49^{th} edn., Medical Economics Data Production Company, Montvale, New Jersey, 1995). In addition, compounds containing more than one of the above-listed components coupled together, *e*.*g*., ranitidine coupled to bismuth subcitrate, may be used.

Amounts of the above-listed compounds used in the methods and compositions of the invention may readily be determined by one skilled in the art. In addition, one skilled the art may readily design treatment/immunization schedules. For example, the non-vaccine components may be administered on days 1-14, and the vaccine antigen + adjuvant may be administered on days 7, 14, 21, and 28.

In addition to urease, other Helicobacter antigens may be used in the methods and compositions of the invention involving combination therapy. For example, *Helicobacter* heat shock proteins A or B (HspA or HspB; WO 94/26901), *Helicobacter* adhesion lipoprotein A (AlpA; The nucleotide sequence encoding AlpA is set forth in SEQ ID NO:6 and the corresponding amino acid sequence of AlpA is in SEQ ID NO:7), *Helicobacter* (*e*.*g*., *H. pylori*) clpB (clpB is encoded by the insert in the plasmid in bacterial strain XLOLR HP CP6, which was deposited with the National Collections of Industrial & Marine Bacteria in Aberdeen, Scotland, and designated with NCIMB accession number 40748), or the 16-19 kD *Helicobacter* antigen recognized by monoclonal antibody IgG 50 (IgG 50 may be obtained from hybridoma cell line #50-G₆-B₇, which was deposited with the ATCC and designated with ATCC accession number HB-11952) may be used. In addition, *Helicobacter* surface-exposed or secreted antigens identified using the transposon shuttle mutagenesis method of Haas *et al*. may be used as vaccine antigens in the invention (Haas et al., Gene 130:23-31, 1993; Haas et al., Abstract from the VIth Workshop on Gastroduodenal Pathology and Helicobacter pylori, Brussels, September 21-25, 1993; Odenbreit et al., Gut, An International Journal of Gastroenterology and Hepatology, Abstract from the VIIIth International Workshop on Gastro-duodenal Pathology and Helicobacter pylori, Edinburgh, Scotland, July 7-9, 1995). In this method, cloned *H. pylori* genes are mutagenized by transposon insertion mutagenesis in *Escherichia coli.* The inactivated genes are reintroduced into *H. pylori* by DNA transformation, and allelic replacement results in the generation of corresponding *H*. *pylori* mutants. The transposon used in one variation of this method carries *blaM*, which is a 5'-truncated version of the β-lactamase gene. Use of this transposon facilitates tagging of genes encoding leader peptide-containing exported proteins, and thus direct selection of clones containing mutants of exported proteins (*e*.*g*., cell surface proteins such as adhesins and cytotoxins, as well as other potential cell surface virulence factors), which are strong candidates for vaccine antigens. In addition to *Helicobacter* antigens, antigens from non-*Helicobacter* gastroduodenal pathogens may be used in methods and compositions for preventing and/or treating infection caused by their respective pathogens. Polypeptide fragments containing protective and/or therapeutic epitopes of the above-listed antigens may also be used in the invention.

Adjuvants which may be used in the methods and compositions of the invention include mucosal adjuvants, *e*.*g*., the heat-labile enterotoxin of *E*. *Coli* (LT), a cholera toxin, a *C*. *difficile* toxin, or derivatives (*e*.*g*., fragments, mutants, or toxoids having adjuvant activity), or combinations thereof. For example, a fragment containing the 794 carboxyl-terminal amino acids of *C.* difficile Toxin A (see, *e*.*g*., Dove et al., *supra,* for the sequence of *C. difficile* Toxin A) may be used. The amounts of vaccine antigen and adjuvant which may be used in these compositions and methods of the invention are described above.

The efficacy of a composition containing a vaccine (*H. pylori* urease + LT), an antibiotic (amoxicillin), and a bismuth salt (pepto-bismol) in the treatment of *Helicobacter* infection is illustrated below.

### Combination therapy for Helicobacter infection of mice

One month after infection with ∼10⁷ *H. felis,* mice were treated with amoxicillin (1.5 mg/30 g mouse) and/or pepto-bismol (0.2 mg/30 g mouse) once per day for 14 days (days 1-14), and/or a vaccine composition containing 100 µg recombinant *H. pylori* urease + 5 µg LT on days 7, 14, 21, and 28 (see Table 9 for an illustration of the combinations used). *Helicobacter* infection in these mice was measured by quantitative gastric urease assay (see above) 1 week and 4 weeks after termination of the treatment (day 28). As is illustrated in Table 9, the most effective treatment regimen, achieving 100% clearance, involved administration of a vaccine (urease + LT), an antibiotic (amoxicillin), and a bismuth salt (pepto-bismol).

**Table 9**

| % Mice Cleared of *H. felis* Infection | | |
|---|---|---|
| treatment weeks | 1 week | 4 |
| amoxicillin | 60% | 40% |
| pepto-bismol | 0% | 0% |
| amoxicillin + pepto-bismol | 80% | 40% |
| vaccine | 70% | 70% |
| vaccine + amoxicillin | 89% | 56% |
| vaccine + pepto-bismol ∼70% | ∼70% | |
| vaccine + amoxicillin + pepto-bismol 100% | 100% | |

| | | |
|---|---|---|
| (vaccine = urease + adjuvant) | | |

### Identification of human patients for administration of recombinant H. pylori urease vaccine

The recombinant *H*. *pylori* urease vaccine of the invention may be administered to uninfected individuals as a prophylactic measure or to individuals infected with *Helicobacter* as an antibacterial therapy. Individuals selected for prophylactic administration of recombinant urease include any individual at risk of *Helicobacter* infection as based upon age, geographical location, or the presence of a condition which renders the individual susceptible to *Helicobacter* infection. Individuals at particularly high risk of infection, or who would be most severely affected by infection, include individuals in developing countries, infants and children in developing and in developed countries, individuals with naturally or artificially low gastric acid pH, submarine crews, and military personnel.

Individuals who may receive the recombinant *H. pylori* urease vaccine as a therapeutic include those individuals with symptoms of gastritis or other gastrointestinal disorders which may be associated with *H. pylori* infection. The clinical symptoms associated with gastritis, an inflammation of the stomach mucosa, include a broad range of poorly-defined, and generally inadequately treated, symptoms such as indigestion, "heart burn," dyspepsia, and excessive eructation. A general discussion of gastritis appears in Sleisenger and Fordtran, In Gastrointestinal Disease, 4th Edn., Saunders Publishing Co., Philadelphia, PA, pp. 772-902, 1989.

Individuals who have a gastrointestinal disorder may also be treated by administration of the vaccine of the invention. Gastrointestinal disorders includes any disease or other disorder of the gastrointestinal tract of a human or other mammal. Gastrointestinal disorders include, for example, disorders not manifested by the presence of ulcerations in the gastric mucosa (non-ulcerative gastrointestinal disorder), including chronic or atrophic gastritis, gastroenteritis, non-ulcer dyspepsia, esophageal reflux disease, gastric motility disorders, and peptic ulcer disease (*e*.*g*., gastric and duodenal ulcers). Peptic ulcers involve ulceration and formation of lesions of the mucous membrane of the esophagus, stomach, or duodenum, and is generally characterized by loss of tissue due to the action of digestive acids, pepsin, or other factors. Alternatively, it may be desirable to administer the vaccine to asymptomatic individuals, particularly where the individual may have been exposed to *H. pylori* or has a condition rendering the individual susceptible to infection.

Infection with *Helicobacter* can be readily diagnosed by a variety of methods well known in the art, including, *e*.*g*., by serology, ¹³C breath test, and/or gastroscopic examination.

### Preparation of purified recombinant urease for administration to patients

The process for formulation of recombinant urease involves combining urease with a stabilizer, (*e*.*g*., a carbohydrate mannitol) and freeze drying (*i*.*e*., lyophilizing) the product. This process prevents degradation by aggregation and fragmentation. In addition, the product is stable for months following lyophilization. The process which leads to instability involves formation of disulfide bonds between protein subunits, and is effectively inhibited by lyophilization.

Recombinant urease is freeze-dried following the final purification step. The purified protein product (approximately 4 mg/ml) is dialyzed against 2% sucrose, and this solution is transferred to lyophilization vials. The vialed solution is either: (1) frozen in liquid nitrogen, and then placed into the lyophilizer, or (2) cooled to 4°C, and then placed in the lyophilizer, where it is frozen to -40°C, or lower. Lyophilization is carried out using standard methods. The freeze-dried product may be reconstituted in water.

### Mode of administration to human patients

Recombinant *H. pylori* urease is administered to a mucosal surface of the individual in order to stimulate a mucosal immune response effective to provide protection to subsequent exposure to *Helicobacter* and/or facilitate clearance of a pre-existing *Helicobacter* infection. Preferably, recombinant urease is administered so as to elicit a mucosal immune response associated with production of anti-urease IgA antibodies and/or infiltration of lymphocytes into the gastric mucosa. The recombinant urease may be administered to any mucosal surface of the patient. Preferable mucosal surfaces are intranasal, oral, and rectal (*e*.*g*., by use of an anal suppository). In addition to being administered to a single mucosal surface, the vaccine of the invention may be administered to combinations of mucosal surfaces (*e*.*g*., oral+rectal, oral+intranasal, or rectal+intranasal). In the case of oral administration, it is preferable that the administration involves ingestion of the vaccine, but the vaccine may also be administered as a mouth wash, so that an immune response is stimulated in the mucosal surface of the oral cavity, without actual ingestion of the vaccine. Alternatively, a systemic mucosal immune response may be achieved by administration of the vaccine to a mucosal surface of the eye in the form of, *e*.*g*., an eye drop or an intraocular implant.

Dosages of recombinant *H. pylori* urease administered to the individual as either a prophylactic therapy or an antibacterial therapy can be determined by one skilled in the art. Generally, dosages will contain between about 10 µg to 1,000 mg, for example, between about 10 mg and 500 mg, 30 mg and 120 mg, 40 mg and 70 mg, or 60 mg recombinant *H. pylori* urease.

At least one dose of the recombinant *H. pylori* urease will be administered to the patient, for example, at least two doses, at least four doses, or with up to six or more total doses administered. It may be desirable to administer booster doses of the recombinant urease at one or two week intervals after the last immunization, generally one booster dose containing less than, or the same amount of, recombinant *H*. *pylori* urease as the initial dose administered. For example, the vaccine regimen may be administered in four doses at one week intervals. The priming and booster doses may be administered to the same or different mucosal surfaces. In the case of different mucosal surfaces, for example, an oral priming dose may be followed by intranasal or rectal boosters, an intranasal priming dose may be followed by oral or rectal boosters, or a rectal priming dose may be followed by oral or intranasal boosters.

Recombinant *H. pylori* urease may be co-administered with a mucosal adjuvant. The mucosal adjuvant may be any mucosal adjuvant known in the art which is appropriate for human use. For example, the mucosal adjuvant may be cholera toxin (CT), enterotoxigenic *E. coli* heat-labile toxin (LT), or a derivative, subunit, or fragment of CT or LT which retains adjuvanticity. The mucosal adjuvant is co-administered with recombinant *H*. *pylori* urease in an amount effective to elicit or enhance a mucosal immune response, particularly a humoral and/or a mucosal immune response. The ratio of adjuvant to recombinant urease may be determined by standard methods by one skilled in the art. For example, the adjuvant may be present at a ratio of 1 part adjuvant to 10 parts recombinant urease.

A buffer may be administered prior to administration of recombinant *H*. *pylori* urease in order to neutralize or increase the pH of the gastric acid. Any buffer that is effective in raising the pH of gastric acid and is appropriate for human use may be used. For example, buffers such as sodium bicarbonate, potassium bicarbonate, and sodium phosphate may be used. In the case of oral administration, the vaccine may be buffer-free, *i*.*e*., no amount of a pH-raising buffer compound effective to significantly affect gastric acid pH is administered to the patient either prior to, or concomitant with, administration of the vaccine recombinant urease.

The vaccine formulation containing recombinant urease may contain a variety of other components, including stabilizers, flavor enhancers (*e*.*g*., sugar), or, where the vaccine is administered as an antibacterial therapeutic, other compounds effective in facilitating clearance and/or eradication of the infecting bacteria.

For prophylactic therapy, the vaccine containing recombinant *H. pylori* urease may be administered at any time prior to contact with, or establishment of, *Helicobacter* infection. Because the vaccine can also act as an antibacterial therapy, there is no contraindication for administration of the vaccine if there is marginal evidence or suspicion of a pre-existing *Helicobacter* infection (*e*.*g*., an asymptomatic infection).

For use of the vaccine as an antibacterial therapy, recombinant *H. pylori* urease may be administered at any time before, during, or after the onset of symptoms associated with *Helicobacter* infection or with gastritis, peptic ulcers or other gastrointestinal disorder. Although it is not a prerequisite to the initiation of therapy, one may confirm diagnosis of *Helicobacter* infection by, *e*.*g*., a ¹³C breath test, serology, gastroscopy, biopsy, or another *Helicobacter* detection method known in the art. The progress of immunized patients may be followed by general medical evaluation, screening for *H. pylori* infection by serology, ¹³C breath test, and/or gastroscopic examination.

### Example of administration of recombinant H. pylori urease to a human

A vaccine composed of 60 mg of recombinant *H*. *pylori* urease in a total volume of 15 ml of water containing 2% weight/volume sucrose, pH 7.5 is orally administered to the patient. Administration of the vaccine is repeated at weekly intervals for a total of 4 doses. Symptoms are recorded daily by the patient. To determine adverse effects, physician interviews are performed weekly during the period of vaccine administration, as well as 1 week and 1 month after the last immunization. Anti-urease antibodies are measured in serum and saliva, and antibody-secreting cells are monitored in peripheral blood collected 7 days after the last immunization.

## Claims

1. A vaccine for inducing a mucosal immune response to *Helicobacter* in a patient, said vaccine comprising:
a) multimeric complexes of recombinant, enzymatically inactive *Helicobacter* urease;
b) a mucosal adjuvant, wherein said mucosal adjuvant is the heat-labile enterotoxin of enterotoxigenic *Escherichia coli*, a cholera toxin, or a fragment or mutant thereof still having adjuvant activity; and
c) a pharmaceutically acceptable carrier or diluent.

2. The vaccine of claim 1, comprising a multimeric complex comprising eight Urease A subunits and eight Urease B subunits, a multimeric complex comprising six Urease A subunits and six Urease B subunits, or a multimeric complex comprising four Urease A subunits and four Urease B subunits.

3. The vaccine of claim 1, comprising a multimeric complex comprising eight Urease A subunits and eight Urease B subunits, a multimeric complex comprising six Urease A subunits and six Urease B subunits, and a multimeric complex comprising four Urease A subunits and four Urease B subunits.

4. The vaccine of claim 1, wherein said mucosal adjuvant is the heat-labile enterotoxin of enterotoxigenic *Escherichia coli*, or a fragment or mutant thereof retaining adjuvant activity.

5. The vaccine of claim 1, wherein said mucosal adjuvant is a cholera toxin, or a fragment or mutant thereof retaining adjuvant activity.

6. The vaccine of claim 1, wherein said multimeric complexes of recombinant, enzymatically inactive *Helicobacter* urease have been freeze-dried.

7. The vaccine of claim 1, wherein said *Helicobacter* urease is *Helicobacter pylori* urease.

8. The vaccine of claim 1, wherein the immune response is protective.

9. The vaccine of claim 1, wherein the immune response is therapeutic.

10. Use of an immunogenically effective amount of multimeric complexes of recombinant, enzymatically inactive *Helicobacter* urease and a mucosal adjuvant, wherein said mucosal adjuvant is the heat-labile enterotoxin of enterotoxigenic *Escherichia coli,* a cholera toxin, or a fragment or mutant thereof still having adjuvant activity, for the preparation of a vaccine for inducing a mucosal immune response to *Helicobacter* in a patient by mucosal administration.

11. The use of claim 10, wherein said multimeric complexes comprise eight Urease A subunits and eight Urease B subunits, six Urease A subunits and six Urease B subunits, or four Urease A subunits and four Urease B subunits.

12. The use of claim 10, wherein said multimeric complexes comprise eight Urease A subunits and eight Urease B subunits, six Urease A subunits and six Urease B subunits, and four Urease A subunits and four Urease B subunits.

13. The use of claim 10, wherein said vaccine is administered to mucosal surfaces, preferably intranasal or oral.

14. The use of claim 10, wherein said vaccine is administered without gastric neutralization.

15. The use of claim 10, wherein said multimeric complexes of recombinant, enzymatically inactive *Helicobacter* urease have been freeze-dried.

16. The use of claim 10, wherein said *Helicobacter* urease is *Helicobacter pylori* urease.

17. The use of claim 10, wherein said *Helicobacter* is *Helicobacter pylori.*

18. The use of claim 10, wherein said patient is at risk of developing, but does not have, a *Helicobacter* infection.

19. The use of claim 10, wherein said patient is infected by *Helicobacter.*

20. The use of claim 10, wherein the immune response is protective.

21. The use of claim 10, wherein the immune response is therapeutic.

## Patentansprüche

1. Vakzine zum Induzieren einer mukosalen Immunantwort gegenüber *Helicobacter* in einem Patienten, wobei die Vakzine:
a) multimere Komplexe von rekombinanter, enzymatisch inaktiver *Helicobacter-Urease,*
b) ein mukosales Adjuvans, wobei das mukosale Adjuvans das hitzelabile Enterotoxin von enterotoxigenen *Escherichia coli,* ein Choleratoxin oder ein Fragment oder eine Mutante davon ist, das/die noch Adjuvansaktivität aufweist, und
c) einen pharmazeutisch verträglichen Träger oder ein pharmazeutisch verträgliches Verdünnungsmittel umfasst.

2. Vakzine nach Anspruch 1, die einen multimeren Komplex, der acht Urease A-Untereinheiten und acht Urease B-Untereinheiten umfasst, einen multimeren Komplex, der sechs Urease A-Untereinheiten und sechs Urease B-Untereinheiten umfasst, oder einen multimeren Komplex umfasst, der vier Urease A-Untereinheiten und vier Urease B-Untereinheiten umfasst.

3. Vakzine nach Anspruch 1, die einen multimeren Komplex, der acht Urease A-Untereinheiten und acht Urease B-Untereinheiten umfasst, einen multimeren Komplex, der sechs Urease A-Untereinheiten und sechs Urease B-Untereinheiten umfasst, und einen multimeren Komplex umfasst, der vier Urease A-Untereinheiten und vier Urease B-Untereinheiten umfasst.

4. Vakzine nach Anspruch 1, wobei das mukosale Adjuvans das hitzelabile Enterotoxin von enterotoxigenen *Escherichia coli* oder ein Fragment oder eine Mutante davon ist, das/die Adjuvansaktivität beibehält.

5. Vakzine nach Anspruch 1, wobei das mukosale Adjuvans ein Choleratoxin oder ein Fragment oder eine Mutante davon ist, das/die Adjuvansaktivität beibehält.

6. Vakzine nach Anspruch 1, wobei die multimeren Komplexe von rekombinanter, enzymatisch inaktiver Helicobacter-Urease gefriergetrocknet wurden.

7. Vakzine nach Anspruch 1, wobei die Helicobacter-Urease Urease von *Helicobacter pylori* ist.

8. Vakzine nach Anspruch 1, wobei die Immunantwort schützend ist.

9. Vakzine nach Anspruch 1, wobei die Immunantwort therapeutisch ist.

10. Verwendung einer immunogen wirksamen Menge von multimeren Komplexen von rekombinanter, enzymatisch inaktiver Helicobacter-Urease und einem mukosalen Adjuvans, wobei das mukosale Adjuvans das hitzelabile Enterotoxin von enterotoxigenen *Escherichia coli*, ein Choleratoxin oder ein Fragment oder eine Mutante davon ist, das/die noch Adjuvansaktivität aufweist, zur Herstellung einer Vakzine zum Induzieren einer mukosalen Immunantwort gegenüber *Helicobacter* in einem Patienten durch mukosale Verabreichung.

11. Verwendung nach Anspruch 10, wobei die multimeren Komplexe acht Urease A-Untereinheiten und acht Urease B-Untereinheiten, sechs Urease A-Untereinheiten und sechs Urease B-Untereinheiten oder vier Urease A-Untereinheiten und vier Urease B-Untereinheiten umfassen.

12. Verwendung nach Anspruch 10, wobei die multimeren Komplexe acht Urease A-Untereinheiten und acht Urease B-Untereinheiten, sechs Urease A-Untereinheiten und sechs Urease B-Untereinheiten und vier Urease A-Untereinheiten und vier Urease B-Untereinheiten umfassen.

13. Verwendung nach Anspruch 10, wobei die Vakzine an mukosale Oberflächen, vorzugsweise intranasal oder oral, verabreicht wird.

14. Verwendung nach Anspruch 10, wobei die Vakzine ohne Neutralisierung des Magens verabreicht wird.

15. Verwendung nach Anspruch 10, wobei die multimeren Komplexe von rekombinanter, enzymatisch inaktiver Helicobacter-Urease gefriergetrocknet wurden.

16. Verwendung nach Anspruch 10, wobei die Helicobacter-Urease Urease von *Helicobacter pylori* ist.

17. Verwendung nach Anspruch 10, wobei *Helicobacter Helicobacter pylori* ist.

18. Verwendung nach Anspruch 10, wobei der Patient ein Risiko aufweist, eine Helicobacter-Infektion zu entwickeln, aber keine aufweist.

19. Verwendung nach Anspruch 10, wobei der Patient mit *Helicobacter* infiziert ist.

20. Verwendung nach Anspruch 10, wobei die Immunantwort schützend ist.

21. Verwendung nach Anspruch 10, wobei die Immunantwort therapeutisch ist.

## Revendications

1. Vaccin pour l'induction d'une réponse immunitaire des muqueuses à *Helicobacter* chez un patient, ledit vaccin comprenant:
a) des complexes multimères d'uréase d'*Helicobacter* recombinante, inactive du point de vue enzymatique;
b) un adjuvant mucosal, tandis que ledit adjuvant mucosal est l'entérotoxine thermolabile d'*Escherichia coli* entérotoxinogène, une toxine cholérique, ou un fragment ou mutant de celles-ci ayant encore une activité d'adjuvant; et
c) un support ou diluant pharmaceutiquement acceptable.

2. Vaccin selon la revendication 1, comprenant un complexe multimère comprenant huit sous-unités Uréase A et huit sous-unités Uréase B, un complexe multimère comprenant six sous-unités Uréase A et six sous-unités Uréase B, ou un complexe multimère comprenant quatre sous-unités Uréase A et quatre sous-unités Uréase B.

3. Vaccin selon la revendication 1, comprenant un complexe multimère comprenant huit sous-unités Uréase A et huit sous-unités Uréase B, un complexe multimère comprenant six sous-unités Uréase A et six sous-unités Uréase B, et un complexe multimère comprenant quatre sous-unités Uréase A et quatre sous-unités Uréase B.

4. Vaccin selon la revendication 1, dans lequel ledit adjuvant mucosal est l'entérotoxine thermolabile d'*Escherichia coli* entérotoxinogène, ou un fragment ou un mutant de celle-ci conservant l'activité d'adjuvant.

5. Vaccin selon la revendication 1, dans lequel ledit adjuvant mucosal est une toxine cholérique, ou un fragment ou un mutant de celle-ci conservant l'activité d'adjuvant.

6. Vaccin selon la revendication 1, dans lequel lesdits complexes multimères d'uréase d'*Helicobacter* recombinante, inactive du point de vue enzymatique ont été lyophilisés.

7. Vaccin selon la revendication 1, dans lequel ladite uréase d'*Helicobacter* est l'uréase d'*Helicobacter pylori*.

8. Vaccin selon la revendication 1, dans lequel la réponse immunitaire est protectrice.

9. Vaccin selon la revendication 1, dans lequel la réponse immunitaire est thérapeutique.

10. Utilisation d'une quantité efficace du point de vue immunogène de complexes multimères d'uréase d'*Helicobacter* recombinante, inactive du point de vue enzymatique et d'un adjuvant mucosal, tandis que ledit adjuvant mucosal est l'entérotoxine thermolabile d'*Escherichia coli* entérotoxinogène, une toxine cholérique, ou un fragment ou un mutant de celles-ci ayant encore une activité d'adjuvant, pour la préparation d'un vaccin pour l'induction d'une réponse immunitaire des muqueuses à *Helicobacter* chez un patient par administration aux muqueuses.

11. Utilisation selon la revendication 10, dans laquelle lesdits complexes multimères comprennent huit sous-unités Uréase A et huit sous-unités Uréase B, six sous-unités Uréase A et six sous-unités Uréase B, ou quatre sous-unités Uréase A et quatre sous-unités Uréase B.

12. Utilisation selon la revendication 10, dans laquelle lesdits complexes multimères comprennent huit sous-unités Uréase A et huit sous-unités Uréase B, six sous-unités Uréase A et six sous-unités Uréase B, et quatre sous-unités Uréase A et quatre sous-unités Uréase B.

13. Utilisation selon la revendication 10, dans laquelle ledit vaccin est administré à des surfaces de muqueuses, de préférence intranasales ou orales.

14. Utilisation selon la revendication 10, dans laquelle ledit vaccin est administré sans neutralisation gastrique.

15. Utilisation selon la revendication 10, dans laquelle lesdits complexes multimères d'uréase d'*Helicobacter* recombinante, inactive du point de vue enzymatique ont été lyophilisés.

16. Utilisation selon la revendication 10, dans laquelle ladite uréase d'*Helicobacter* est l'uréase d'*Helicobacter pylori*.

17. Utilisation selon la revendication 10, dans laquelle ledit *Helicobacter* est *Helicobacter pylori*.

18. Utilisation selon la revendication 10, dans laquelle ledit patient est sujet à un risque de développer, mais n'a pas, une infection par *Helicobacter.*

19. Utilisation selon la revendication 10, dans laquelle ledit patient est infecté par *Helicobacter.*

20. Utilisation selon la revendication 10, dans laquelle la réponse immunitaire est protectrice.

21. Utilisation selon la revendication 10, dans laquelle la réponse immunitaire est thérapeutique.


### SEQUENCE LISTING

(1) GENERAL INFORMATION:
   (i) APPLICANT: OraVax, Inc.
   (ii) TITLE OF INVENTION: MULTIMERIC, RECOMBINANT UREASE VACCINE
   (iii) NUMBER OF SEQUENCES: 7
   (iv) CORRESPONDENCE ADDRESS:
      (A) ADDRESSEE: Fish & Richardson P.C.
      (B) STREET: 225 Franklin Street
      (C) CITY: Boston
      (D) STATE: MA
      (E) COUNTRY: USA
      (F) ZIP: 02110-2804
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT/US96/- - - -
      (B) FILING DATE: 23-APR-1996
      (C) CLASSIFICATION:
   (vii) PRIOR APPLICATION DATA:
      (A) APPLICATION NUMBER: 08/431,041
      (B) FILING DATE: 28-APR-1995
      (C) CLASSIFICATION
   (vii) PRIOR APPLICATION DATA:
      (A) APPLICATION NUMBER: 08/568,122
      (B) FILING DATE: 06-DEC-1995
      (C) CLASSIFICATION
   (viii) ATTORNEY/AGENT INFORMATION:
      (A) NAME: Clark, Paul T.
      (B) REGISTRATION NUMBER: 30,162
      (C) REFERENCE/DOCKET NUMBER: 06132/020001
   (ix) TELECOMMUNICATION INFORMATION:
      (A) TELEPHONE: (617) 542-5070
      (B) TELEFAX: (617) 542-8906
(2) INFORMATION FOR SEQ ID NO:1:
   (i) SEQUENCE CHARACTERISTICS:
      (A) LENGTH: 2735 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:1:
   TAATACGACT CACTATAGGG GAATTGTGAG CGGATAACAA TTCATCCACC TTGATTGCGT      60
   TATGTCTTCA AGGAAAAACA CTTTAAGAAT AGGAGAATGA GATGAAACTC ACCCCAAAAG     120
   AGTTAGATAA GTTGATGCTC CACTACGCTG GAGAATTGGC TAAAAAACGC AAAGAAAAAG 180
   GCATTAAGCT TAACTATGTA GAAGCAGTAG CTTTGATTAG TGCCCATATT ATGGAAGAAG 240
   CGAGAGCTGG TAAAAAGACT GCGGCTGAAT TGATGCAAGA AGGGCGCACT CTTTTAAAAC 300
   CAGATGATGT GATGGATGGC GTGGCAAGCA TGATCCATGA AGTGGGTATT GAAGCGATGT 360
   TTCCTGATGG GACTAAACTC GTAACCGTGC ATACCCCTAT TGAGGCCAAT GGTAAATTAG 420
   TTCCTGGTGA GTTGTTCTTA AAAAATGAAG ACATCACTAT CAACGAAGGC AAAAAAGCCG 480
   TTAGCGTGAA AGTTAAAAAT GTTGGCGACA GACCGGTTCA AATCGGCTCA CACTTCCATT 540
   TCTTTGAAGT GAATAGATGC CTAGACTTTG ACAGAGAAAA AACTTTCGGT AAACGCTTAG 600
   ACATTGCGAG CGGGACAGCG GTAAGATTTG AGCCTGGCGA AGAAAAATCC GTAGAATTGA 660
   TTGACATTGG CGGTAACAGA AGAATCTTTG GATTTAACGC ATTGGTTGAT AGACAAGCAG 720
   ACAACGAAAG CAAAAAAATT GCTTTACACA GAGCTAAAGA GCGTGGTTTT CATGGCGCTA 780
   AAAGCGATGA CAACTATGTA AAAACAATTA AGGAGTAAGA AATGAAAAAG ATTAGCAGAA 840
   AAGAATATGT TTCTATGTAT GGTCCTACTA CAGGCGATAA AGTGAGATTG GGCGATACAG 900
   ACTTGATCGC TGAAGTAGAA CATGACTACA CCATTTATGG CGAAGAGCTT AAATTCGGTG 960
   GCGGTAAAAC CCTAAGAGAA GGCATGAGCC AATCTAACAA CCCTAGCAAA GAAGAGTTGG 1020
   ATTTAATTAT CACTAACGCT TTAATCGTGG ATTACACCGG TATTTATAAA GCGGATATTG 1080
   GTATTAAAGA TGGCAAAATC GCTGGCATTG GTAAAGGCGG TAACAAAGAC ATGCAAGATG 1140
   GCGTTAAAAA CAATCTTAGC GTAGGTCCTG CTACTGAAGC CTTAGCCGGT GAAGGTTTGA 1200
   TCGTAACGGC TGGTGGTATT GACACACACA TCCACTTCAT TTCACCCCAA CAAATCCCTA 1260
   CAGCTTTTGC AAGCGGTGTA ACAACCATGA TTGGTGGTGG AACCGGTCCT GCTGATGGCA 1320
   CTAATGCGAC TACTATCACT CCAGGCAGAA GAAATTTAAA ATGGATGCTC AGAGCGGCTG 1380
   AAGAATATTC TATGAATTTA GGTTTCTTGG CTAAAGGTAA CGCTTCTAAC GATGCGAGCT 1440
   TAGCCGATCA AATTGAAGCC GGTGCGATTG GCTTTAAAAT TCACGAAGAC TGGGGCACCA 1500
   CTCCTTCTGC AATCAATCAT GCGTTAGATG TTGCGGACAA ATACGATGTG CAAGTCGCTA 1560
   TCCACACAGA CACTTTGAAT GAAGCCGGTT GTGTAGAAGA CACTATGGCT GCTATTGCTG 1620
   GACGCACTAT GCACACTTTC CACACTGAAG GCGCTGGCGG CGGACACGCT CCTGATATTA 1680
   TTAAAGTAGC CGGTGAACAC AACATTCTTC CCGCTTCCAC TAACCCCACC ATCCCTTTCA 1740
   CCGTGAATAC AGAAGCAGAG CACATGGACA TGCTTATGGT GTGCCACCAC TTGGATAAAA 1800
   GCATTAAAGA AGATGTTCAG TTCGCTGATT CAAGGATCCG CCCTCAAACC ATTGCGGCTG 1860
   AAGACACTTT GCATGACATG GGGATTTTCT CAATCACCAG TTCTGACTCT CAAGCGATGG 1920
   GCCGTGTGGG TGAAGTTATC ACTAGAACTT GGCAAACAGC TGACAAAAAC AAGAAAGAAT 1980
   TTGGCCGCTT GAAAGAAGAA AAAGGCGATA ACGACAACTT CAGGATCAAA CGCTACTTGT 2040
   CTAAATACAC CATTAACCCA GCGATCGCTC ATGGGATTAG CGAGTATGTA GGTTCAGTAG    2100
   AAGTGGGCAA AGTGGCTGAC TTGGTATTGT GGAGTCCAGC ATTCTTTGGC GTGAAACCCA    2160
   ACATGATCAT CAAAGGCGGA TTCATTGCGT TAAGCCAAAT GGGCGATGCG AACGCTTCTA    2220
   TCCCTACCCC ACAACCGGTT TATTACAGAG AAATGTTCGC TCATCATGGT AAAGCTAAAT    2280
   ACGATGCAAA CATCACTTTT GTGTCTCAAG CGGCTTATGA CAAAGGCATT AAAGAAGAAT    2340
   TAGGACTTGA AAGACAAGTG TTGCCGGTAA AAAATTGCAG AAATATCACT AAAAAAGACA    2400
   TGCAATTCAA CGACACTACT GCTCACATTG AAGTCAATCC TGAAACTTAC CATGTGTTCG    2460
   TGGATGGCAA AGAAGTAACT TCTAAACCAG CCAATAAAGT GAGCTTGGCG CAACTCTTTA    2520
   GCATTTTCTA GGATTTTTTA GGAGCAACGC TTCCTTAAAT CCTGAATTCG AGCTCCGTCG    2580
   ACAAGCTTGC GGCCGCACTC GAGCACCACC ACCACCACCA CTGAGATCCG GCTGCTAACA    2640
   AAGCCCGAAA GGAAGCTGAG TTGGCTGCTG CCACCGCTGA GCAATAACTA GCATAACCCC    2700
   TTGGGGCCTC TAAACGGGTC TTGAGGGGTT TTTTG                               2735
(2) INFORMATION FOR SEQ ID NO:2:
   (i) SEQUENCE CHARACTERISTICS:
      (A) LENGTH: 238 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Leu Thr Pro Lys Glu Leu Asp Lys Leu Met Leu His Tyr Ala
   1               5                   10                  15
   Gly Glu Leu Ala Lys Lys Arg Lys Glu Lys Gly Ile Lys Leu Asn Tyr
               20                  25                  30
   Val Glu Ala Val Ala Leu Ile Ser Ala His Ile Met Glu Glu Ala Arg
           35                  40                  45
   Ala Gly Lys Lys Thr Ala Ala Glu Leu Met Gln Glu Gly Arg Thr Leu
       50                  55                  60
   Leu Lys Pro Asp Asp Val Met Asp Gly Val Ala Ser Met Ile His Glu
   65                  70                  75                  80
   Val Gly Ile Glu Ala Met Phe Pro Asp Gly Thr Lys Leu Val Thr Val
                   85                  90                  95
   His Thr Pro Ile Glu Ala Asn Gly Lys Leu Val Pro Gly Glu Leu Phe
               100                 105                 110
   Leu Lys Asn Glu Asp Ile Thr Ile Asn Glu Gly Lys Lys Ala Val Ser
           115                 120                 125
   Val Lys Val Lys Asn Val Gly Asp Arg Pro Val Gln Ile Gly Ser His
       130                 135                 140
   Phe His Phe Phe Glu Val Asn Arg Cys Leu Asp Phe Asp Arg Glu Lys
   145 150 155 160
   Thr Phe Gly Lys Arg Leu Asp Ile Ala Ser Gly Thr Ala Val Arg Phe
   165 170 175
   Glu Pro Gly Glu Glu Lys Ser Val Glu Leu Ile Asp Ile Gly Gly Asn
   180 185 190
   Arg Arg Ile Phe Gly Phe Asn Ala Leu Val Asp Arg Gln Ala Asp Asn
   195 200 205
   Glu Ser Lys Lys Ile Ala Leu His Arg Ala Lys Glu Arg Gly Phe His
   210 215 220
   Gly Ala Lys Ser Asp Asp Asn Tyr Val Lys Thr Ile Lys Glu
   225 230 235
(2) INFORMATION FOR SEQ ID NO:3:
   (i) SEQUENCE CHARACTERISTICS:
      (A) LENGTH: 566 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Met Lys Lys Ile Ser Arg Lys Glu Tyr Val Ser Met Tyr Gly Pro Thr
   1 5 10 15
   Thr Gly Asp Lys Val Arg Leu Gly Asp Thr Asp Leu Ile Ala Glu Val
   20 25 30
   Glu His Asp Tyr Thr Ile Tyr Gly Glu Glu Leu Lys Phe Gly Gly Gly
   35 40 45
   Lys Thr Leu Arg Glu Gly Met Ser Gln Ser Asn Asn Pro Ser Lys Glu
   50 55 60
   Glu Leu Asp Leu Ile Ile Thr Asn Ala Leu Ile Val Asp Tyr Thr Gly
   65 70 75 80
   Ile Tyr Lys Ala Asp Ile Gly Ile Lys Asp Gly Lys Ile Ala Gly Ile
   85 90 95
   Gly Lys Gly Gly Asn Lys Asp Met Gln Asp Gly Val Lys Asn Asn Leu
   100 105 110
   Ser Val Gly Pro Ala Thr Glu Ala Leu Ala Gly Glu Gly Leu Ile Val
   115 120 125
   Thr Ala Gly Gly Ile Asp Thr His Ile His Phe Ile Ser Pro Gln Gln
   130 135 140
   Ile Pro Thr Ala Phe Ala Ser Gly Val Thr Thr Met Ile Gly Gly Gly
   145 150 155 160
   Thr Gly Pro Ala Asp Gly Thr Asn Ala Thr Thr Ile Thr Pro Gly Arg
   165 170 175
   Arg Asn Leu Lys Trp Met Leu Arg Ala Ala Glu Glu Tyr Ser Met Asn
               180                 185                 190
   Leu Gly Phe Leu Ala Lys Gly Asn Ala Ser Asn Asp Ala Ser Leu Ala
           195                 200                 205
   Asp Gln Ile Glu Ala Gly Ala Ile Gly Phe Lys Ile His Glu Asp Trp
       210                 215                 220
   Gly Thr Thr Pro Ser Ala Ile Asn His Ala Leu Asp Val Ala Asp Lys
   225                 230                 235                 240
   Tyr Asp Val Gln Val Ala Ile His Thr Asp Thr Leu Asn Glu Ala Gly
                   245                 250                 255
   Cys Val Glu Asp Thr Met Ala Ala Ile Ala Gly Arg Thr Met His Thr
               260                 265                 270
   Phe His Thr Glu Gly Ala Gly Gly Gly His Ala Pro Asp Ile Ile Lys
           275                 280                 285
   Val Ala Gly Glu His Asn Ile Leu Pro Ala Ser Thr Asn Pro Thr Ile
       290                 295                 300
   Pro Phe Thr Val Asn Thr Glu Ala Glu His Met Asp Met Leu Met Val
   305                 310                 315                 320
   Cys His His Leu Asp Lys Ser Ile Lys Glu Asp Val Gln Phe Ala Asp
                   325                 330                 335
   Ser Arg Ile Arg Pro Gln Thr Ile Ala Ala Glu Asp Thr Leu His Asp
               340                 345                 350
   Met Gly Ile Phe Ser Ile Thr Ser Ser Asp Ser Gln Ala Met Gly Arg
           355                 360                 365
   Val Gly Glu Val Ile Thr Arg Thr Trp Gln Thr Ala Asp Lys Asn Lys
       370                 375                 380
   Lys Glu Phe Gly Arg Leu Lys Glu Glu Lys Gly Asp Asn Asp Asn Phe
   385                 390                 395                 400
   Arg Ile Lys Arg Tyr Leu Ser Lys Tyr Thr Ile Asn Pro Ala Ile Ala
                   405                 410                 415
   His Gly Ile Ser Glu Tyr Val Gly Ser Val Glu Val Gly Lys Val Ala
               420                 425                 430
   Asp Leu Val Leu Trp Ser Pro Ala Phe Phe Gly Val Lys Pro Asn Met
           435                 440                 445
   Ile Ile Lys Gly Gly Phe Ile Ala Leu Ser Gln Met Gly Asp Ala Asn
       450                 455                 460
   Ala Ser Ile Pro Thr Pro Gln Pro Val Tyr Tyr Arg Glu Met Phe Ala
   465                 470                 475                 480
   His His Gly Lys Ala Lys Tyr Asp Ala Asn Ile Thr Phe Val Ser Gln
                   485                 490                 495
   Ala Ala Tyr Asp Lys Gly Ile Lys Glu Glu Leu Gly Leu Glu Arg Gln
               500                 505                 510
   Val Leu Pro Val Lys Asn Cys Arg Asn Ile Thr Lys Lys Asp Met Gln
           515                 520                 525
   Phe Asn Asp Thr Thr Ala His Ile Glu Val Asn Pro Glu Thr Tyr His
       530                 535                 540
   Val Phe Val Asp Gly Lys Glu Val Thr Ser Lys Pro Ala Asn Lys Val
   545                 550                 555                 560
   Ser Leu Ala Gln Leu Phe
                   565
(2) INFORMATION FOR SEQ ID NO:4:
   (i) SEQUENCE CHARACTERISTICS:
      (A) LENGTH: 28 base pairs
      (B) TYPE: nucleic acid
      (C) STRANDEDNESS: both
      (D) TOPOLOGY: both
   (ii) MOLECULE TYPE: cDNA
   (xi) SEQUENCE DESCRIPTION: SEQ ID NO:4:
      CGGGATCCAC CTTGATTGCG TTATGTCT 28
(2) INFORMATION FOR SEQ ID HO:5:
   (i) SEQUENCE CHARACTERISTICS:
      (A) LENGTH: 27 base pairs
      (B) TYPE: nucleic acid
      (C) STRANDEDNESS: both
      (D) TOPOLOGY: both
   (ii) MOLECULE TYPE: cDNA
   (xi) SEQUENCE DESCRIPTION: SEQ ID NO:5:
      CGGAATTCAG GATTTAAGGA AGCGTTG 27
(2) INFORMATION FOR SEQ ID NO:6:
   (i) SEQUENCE CHARACTERISTICS:
      (A) LENGTH: 1557 base pairs
      (B) TYPE: nucleic acid
      (C) STRANDEDNESS: both
      (D) TOPOLOGY: both
   (ii) MOLECULE TYPE: cDNA
   (xi) SEQUENCE DESCRIPTION: SEQ ID NO:6:
   ATGATAAAAA AGAATAGAAC GCTGTTTCTT AGTCTAGCCC TTTGCGCTAG CATAAGTTAT    60
   GCCGAAGATG ATGGAGGGTT TTTCACCGTC GGTTATCAGC TCGGGCAAGT CATGCAAGAT   120
   GTCCAAAACC CAGGCGGCGC TAAAAGCGAC GAACTCGCCA GAGAGCTTAA CGCTGATGTA   180
   ACGAACAACA TTTTAAACAA CAACACCGGA GGCAACATCG CAGGGGCGTT GAGTAACGCT   240
   TTCTCCCAAT ACCTTTATTC GCTTTTAGGG GCTTACCCCA CAAAACTCAA TGGTAGCGAT   300
   GTGTCTGCGA ACGCTCTTTT AAGTGGTGCG GTAGGCTCTG GGACTTGTGC GGCTGCAGGG   360
   ACGGCTGGTG GCACTTCTCT TAACACTCAA AGCACTTGCA CCGTTGCGGG CTATTACTGG    420
   CTCCCTAGCT TGACTGACAG GATTTTAAGC ACGATCGGCA GCCAGACTAA CTACGGCACG    480
   AACACCAATT TCCCCAACAT GCAACAACAG CTCACCTACT TGAATGCGGG GAATGTGTTT    540
   TTTAATGCGA TGAATAAGGC TTTAGAGAAT AAGAATGGAA CTAGTAGTGC TAGTGGAACT    600
   AGTGGTGCGA CTGGTTCAGA TGGTCAAACT TACTCCACAC AAGCTATCCA ATACCTTCAA    660
   GGCCAACAAA ATATCTTAAA TAACGCAGCG AACTTGCTCA AGCAAGATGA ATTGCTCTTA    720
   GAAGCTTTCA ACTCTGCCGT AGCCGCCAAC ATTGGGAATA AGGAATTCAA TTCAGCCGCT    780
   TTTACAGGTT TGGTGCAAGG CATTATTGAT CAATCTCAAG CGGTTTATAA CGAGCTCACT    840
   AAAAACACCA TTAGCGGGAG TGCGGTTATT AGCGCTGGGA TAAACTCCAA CCAAGCTAAC    900
   GCTGTGCAAG GGCGCGCTAG TCAGCTCCCT AACGCTCTTT ATAACGCGCA AGTAACTTTG    960
   GATAAAATCA ATGCGCTCAA TAATCAAGTG AGAAGCATGC CTTACTTGCC CCAATTCAGA   1020
   GCCGGGAACA GCCGTTCAAC GAATATTTTA AACGGGTTTT ACACCAAAAT AGGCTATAAG   1080
   CAATTCTTCG GGAAGAAAAG GAATATCGGT TTGCGCTATT ATGGTTTCTT TTCTTATAAC   1140
   GGAGCGAGCG TGGGCTTTAG ATCCACTCAA AATAATGTAG GGTTATACAC TTATGGGGTG   1200
   GGGACTGATG TGTTGTATAA CATCTTTAGC CGCTCCTATC AAAACCGCTC TGTGGATATG   1260
   GGCTTTTTTA GCGGTATCCA ATTAGCCGGT GAGACCTTCC AATCCACGCT CAGAGATGAC   1320
   CCCAATGTGA AATTGCATGG GAAAATCAAT AACACGCACT TCCAGTTCCT CTTTGACTTC   1380
   GGTATGAGGA TGAACTTCGG TAAGTTGGAC GGGAAATCCA ACCGCCACAA CCAGCACACG   1440
   GTGGAATTTG GCGTAGTGGT GCCTACGATT TATAACACTT ATTACAAATC AGCAGGGACT   1500
   ACCGTGAAGT ATTTCCGTCC TTATAGCGTT TATTGGTCTT ATGGGTATTC ATTCTAA      1557
(2) INFORMATION FOR SEQ ID NO:7:
   (i) SEQUENCE CHARACTERISTICS:
      (A) LENGTH: 518 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Ile Lys Lys Asn Arg Thr Leu Phe Leu Ser Leu Ala Leu Cys Ala
    1               5                  10                  15
   Ser Ile Ser Tyr Ala Glu Asp Asp Gly Gly Phe Phe Thr Val Gly Tyr
               20                  25                  30
   Gln Leu Gly Gln Val Met Gln Asp Val Gln Asn Pro Gly Gly Ala Lys
           35                  40                  45
   Ser Asp Glu Leu Ala Arg Glu Leu Asn Ala Asp Val Thr Asn Asn Ile
       50                  55                  60
   Leu Asn Asn Asn Thr Gly Gly Asn Ile Ala Gly Ala Leu Ser Asn Ala
   65 70 75 80
   Phe Ser Gln Tyr Leu Tyr Ser Leu Leu Gly Ala Tyr Pro Thr Lys Leu
   85 90 95
   Asn Gly Ser Asp Val Ser Ala Asn Ala Leu Leu Ser Gly Ala Val Gly
   100 105 110
   Ser Gly Thr Cys Ala Ala Ala Gly Thr Ala Gly Gly Thr Ser Leu Asn
   115 120 125
   Thr Gln Ser Thr Cys Thr Val Ala Gly Tyr Tyr Trp Leu Pro Ser Leu
   130 135 140
   Thr Asp Arg Ile Leu Ser Thr Ile Gly Ser Gln Thr Asn Tyr Gly Thr
   145 150 155 160
   Asn Thr Asn Phe Pro Asn Met Gln Gln Gln Leu Thr Tyr Leu Asn Ala
   165 170 175
   Gly Asn Val Phe Phe Asn Ala Met Asn Lys Ala Leu Glu Asn Lys Asn
   180 185 190
   Gly Thr Ser Ser Ala Ser Gly Thr Ser Gly Ala Thr Gly Ser Asp Gly
   195 200 205
   Gln Thr Tyr Ser Thr Gln Ala Ile Gln Tyr Leu Gln Gly Gln Gln Asn
   210 215 220
   Ile Leu Asn Asn Ala Ala Asn Leu Leu Lys Gln Asp Glu Leu Leu Leu
   225 230 235 240
   Glu Ala Phe Asn Ser Ala Val Ala Ala Asn Ile Gly Asn Lys Glu Phe
   245 250 255
   Asn Ser Ala Ala Phe Thr Gly Leu Val Gln Gly Ile Ile Asp Gln Ser
   260 265 270
   Gln Ala Val Tyr Asn Glu Leu Thr Lys Asn Thr Ile Ser Gly Ser Ala
   275 280 285
   Val Ile Ser Ala Gly Ile Asn Ser Asn Gln Ala Asn Ala Val Gln Gly
   290 295 300
   Arg Ala Ser Gln Leu Pro Asn Ala Leu Tyr Asn Ala Gln Val Thr Leu
   305 310 315 320
   Asp Lys Ile Asn Ala Leu Asn Asn Gln Val Arg Ser Met Pro Tyr Leu
   325 330 335
   Pro Gln Phe Arg Ala Gly Asn Ser Arg Ser Thr Asn Ile Leu Asn Gly
   340 345 350
   Phe Tyr Thr Lys Ile Gly Tyr Lys Gln Phe Phe Gly Lys Lys Arg Asn
   355 360 365
   Ile Gly Leu Arg Tyr Tyr Gly Phe Phe Ser Tyr Asn Gly Ala Ser Val
   370 375 380
   Gly Phe Arg Ser Thr Gln Asn Asn Val Gly Leu Tyr Thr Tyr Gly Val
   385 390 395 400
   Gly Thr Asp Val Leu Tyr Asn Ile Phe Ser Arg Ser Tyr Gln Asn Arg
   405 410 415
   Ser Val Asp Met Gly Phe Phe Ser Gly Ile Gln Leu Ala Gly Glu Thr
   420 425 430
   Phe Gln Ser Thr Leu Arg Asp Asp Pro Asn Val Lys Leu His Gly Lys
   435 440 445
   Ile Asn Asn Thr His Phe Gln Phe Leu Phe Asp Phe Gly Met Arg Met
   450 455 460
   Asn Phe Gly Lys Leu Asp Gly Lys Ser Asn Arg His Asn Gln His Thr
   465 470 475 480
   Val Glu Phe Gly Val Val Val Pro Thr Ile Tyr Asn Thr Tyr Tyr Lys
   485 490 495
   Ser Ala Gly Thr Thr Val Lys Tyr Phe Arg Pro Tyr Ser Val Tyr Trp
   500 505 510
   Ser Tyr Gly Tyr Ser Phe
   515